# EUROPÄISCHE PATENTANMELDUNG

(11) **EP 0 854 147 A2**
(43) Veröffentlichungstag der Anmeldung: **22.07.1998**
(21) Anmeldenummer: 98100436.9
(22) Anmeldetag: 13.01.1998
(51) Int. Cl.: C07F 7/00

(54) **Tripodale Cyclopentadienderivate und deren Verwendung**

(30) Priorität: 21.01.1997 DE 19701866
(71) Anmelder: BASF AKTIENGESELLSCHAFT, 67056 Ludwigshafen (DE)
(72) Erfinder: Huttner, Gottfried, Prof. Dr., 69115 Heidelberg (DE); Vogelgesang, Joachim, 68219 Mannheim (DE); Winterhalter, Ute, 69190 Walldorf (DE); Antelmann, Björn, Dr., 29227 Celle (DE)

(57) **Zusammenfassung**

Tripodale Cyclopentadienderivate der allgemeinen Formel (I) 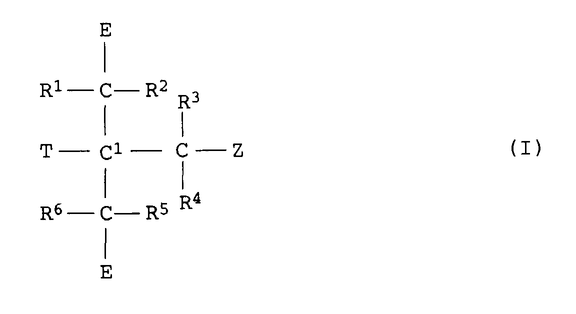 worin
- E: gleich oder verschieden ist und für -N(R) (R), -P(R) (R), -As(R) (R), -Sb(R) (R), -OR, -SR, -SeR, -TeR, wobei R gleich oder verschieden ist und Wasserstoff- einen C₁- bis C₂₀-kohlenstofforganischen oder C₁- bis C₃₀-siliciumorganischen Rest bedeutet, oder E für eine Abgangsgruppe X steht und
- R¹, R², R³, R⁴, R⁵, R⁶: gleich oder verschieden ist und Wasserstoff einen C₁- bis C_{2O}- kohlenstofforganischen oder C₁- bis C_{3O}-siliciumorganischen Rest bedeutet,
- Z: für den Cyclopentadienylrest oder eine substituierte Cyclopentadienylstruktureinheit steht und
- T: Wasserstoff einen C₁- bis C₂₀-kohlenstofforganischen oder C₁- bis C₃₀-siliciumorganischen Rest bedeutet oder für eine Gruppe E-Y- steht, worin E für -N(R) (R), -P(R) (R), -As(R) (R), -Sb(R) (R), -OR, -SR, -SeR, -TeR oder eine Abgangsgruppe X steht, wobei R gleich oder verschieden ist und Wasserstoff einen C₁- bis C_{2O}-kohlenstofforganischen oder C₁- bis C_{3O}-siliciumorganischen Rest bedeutet und worin Y eine C₁- bis C_{2O}-organische Gruppe ist, die E mit C¹ verbindet.

## Beschreibung

Die vorliegende Erfindung betrifft tripodale Cyclopentadienderivate der allgemeinen Formel (I) 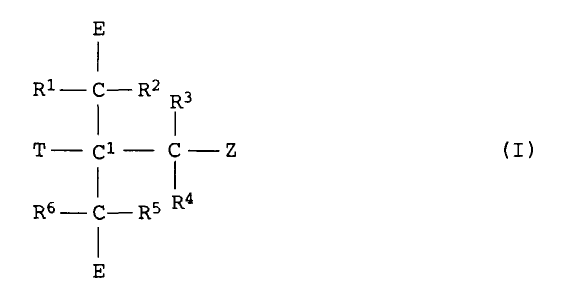 worin
- E: gleich oder verschieden ist und für -N(R) (R), -P(R) (R), -As(R) (R), -Sb(R) (R), -OR, -SR, -SeR, -TeR, wobei R gleich oder verschieden ist und Wasserstoff- einen C₁- bis C₂₀-kohlenstofforganischen oder C₁- bis C₃₀-siliciumorganischen Rest bedeutet, oder E für eine Abgangsgruppe X steht und
- R¹, R², R³, R⁴, R⁵, R⁶: gleich oder verschieden ist und Wasserstoff einen C₁- bis C_{2O}- kohlenstofforganischen oder C₁- bis C_{3O}-siliciumorganischen Rest bedeutet,
- Z: für den Cyclopentadienylrest oder eine substituierte Cyclopentadienylstruktureinheit steht und
- T: Wasserstoff einen C₁- bis C₂₀-kohlenstofforganischen oder C₁- bis C₃₀-siliciumorganischen Rest bedeutet oder für eine Gruppe E-Y- steht, worin E für -N(R) (R), -P(R) (R), -As(R) (R), -Sb(R) (R), -OR, -SR, -SeR, -TeR oder eine Abgangsgruppe X steht, wobei R gleich oder verschieden ist und Wasserstoff einen C₁- bis C_{2O}-kohlenstofforganischen oder C₁- bis C_{3O}- siliciumorganischen Rest bedeutet und worin Y eine C₁- bis C_{2O}-organische Gruppe ist, die E mit C¹ verbindet.

Weiterhin betrifft die vorliegende Erfindung Verfahren zur Herstellung von tripodalen Cyclopentadienderivaten (I), sowie die Verwendung der Verbindungen (I) als Liganden in Metallkomplexen, ferner Tripodmetallkomplexe der allgemeinen Formel (V)

LₙM(Tₚ)ₘ (V)

worin
- M: ein Übergangsmetall oder ein Hauptgruppenmetall des Periodensystems der Elemente
- Tp: ein einfach deprotoniertes Cyclopentadienderivat der allgemeinen Formel (I) 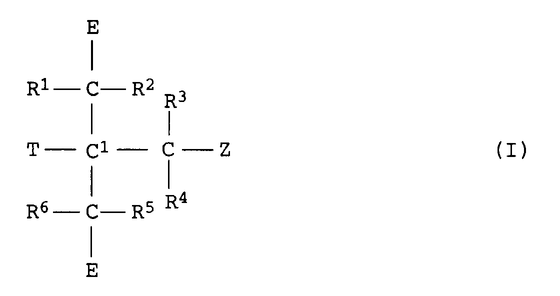
worin
- E: gleich oder verschieden ist und für -N(R) (R), -P(R) (R), -As(R) (R), -Sb(R) (R), -OR, -SR, -SeR, -TeR, wobei R gleich oder verschieden ist und Wasserstoff- einen C₁- bis C₂₀-kohlenstofforganischen oder C₁- bis C₃₀-siliciumorganischen Rest bedeutet, oder E für eine Abgangsgruppe X steht und
- R¹, R², R³, R⁴, R⁵, R⁶: gleich oder verschieden ist und Wasserstoff einen C₁- bis C_{2O}- kohlenstofforganischen oder C₁- bis C_{3O}-siliciumorganischen Rest bedeutet,
- Z: für den Cyclopentadienylrest oder eine substituierte Cyclopentadienylstruktureinheit steht und
- T: Wasserstoff einen C₁- bis C₂₀-kohlenstofforganischen oder C₁- bis C₃₀-siliciumorganischen Rest bedeutet oder für eine Gruppe E-Y- steht, worin E für -N(R) (R), -P(R) (R), -As(R) (R), -Sb(R) (R), -OR, -SR, -SeR, -TeR oder eine Abgangsgruppe X steht, wobei R gleich oder verschieden ist und Wasserstoff einen C₁- bis C_{2O}-kohlenstofforganischen oder C₁- bis C_{3O}-siliciumorganischen Rest bedeutet und worin Y eine C₁- bis C_{2O}-organische Gruppe ist, die E mit C¹ verbindet.
- L: ein formal anionischer oder neutraler Ligand, oder gleiche oder verschiedene derartige Liganden
- n: eine ganze Zahl von 0 bis 7,
- m: eine ganze Zahl von 1 bis 8
bedeutet und die Verwendung dieser Komplexe zur stöchiometrischen oder katalytischen Kohlenstoff-Kohlenstoff-Bindungsverknüpfung oder Hydrierung.

Substituierte Cyclopentadienylverbindungen stellen wichtige Liganden in der Übergangsmetallkatalyse dar. Ein Beispiel aus der Polymerchemie sind die sogenannten Metallocenkatalysatoren, H. H. Brintzinger, D. Fischer et al., Angew. Chem. (1995), Seite 1255 bis 1283.

Die Struktur und chemische Konstitution der Liganden haben großen Einfluß auf die katalytischen Eigenschaften der mit ihnen erhältlichen Metallkomplexe.

Aus J. A. Ewen et al., Makromol. Chem. Makromol. Symp. 48/49 (1991), S. 253-295 ist beispielsweise bekannt, daß die Ligandenstruktur in Metallocenen (Komplexe von Metallen mit mindestens einem Cyclopentadienyltyp-Liganden), die Bestandteile von Katalysatoren für die Olefinpolymerisation sind, Einfluß auf die Polymereigenschaften nehmen kann.

Da die Technik nach neuen, besseren Kunststoffen, Wirkstoffen und Effektstoffen verlangt, die mit bekannten Liganden- und Katalysatorsystemen nur eingeschränkt erhalten werden können, besteht die Herausforderung neue, als Liganden in katalytisch aktiven Metallkomplexen geeignete, Verbindungen herzustellen.

Aufgabe der vorliegenden Erfindung war es daher neue Cyclopentadienderivate zur Verfügung zu stellen, die noch Donorzentren aufweisen, welche eine zur Cyclopentadienstruktur unterschiedliche Struktur haben und die gegebenenfalls über eine weitere Funktionalität (Ankergruppe oder lyophilisierende Gruppe), beispielsweise an Trägermaterial, physikalisch oder insbesondere chemisch fixierbar sind und die als Liganden in Metallkomplexen geeignet sind.

Aufgabe der vorliegenden Erfindung war es weiterhin chirale Verbindungen zur Verfügung zu stellen, die als Liganden für chirale, katalytisch aktive Metallkomplexe, oder chirale Metallkomplexe in Katalysatorsystemen verwendbar sind.

Demgemäß wurden die eingangs definierten tripodalen Cyclopentadienderivate (I), Verfahren zu deren Herstellung, sowie die Verwendung von (I) als Liganden in Metallkomplexen oder Katalysatorsystemen sowie Tripodmetallkomplexe (V) und deren Verwendung zur stöchiometrischen oder katalytischen Kohlenstoff-Kohlenstoff-Bindungsverknüpfung oder Hydrierung gefunden.

Die Substituenten E in (I) sind entweder gleich, oder verschieden. Sie stehen für 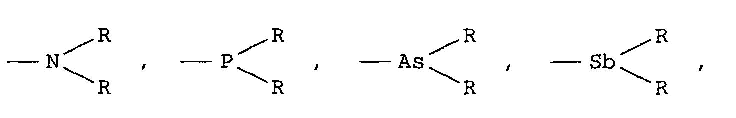 -OR, -SR, -SeR oder -TeR, oder eine Abgangsgruppe X wie Chlor, Brom, -OSO₂CH₃, OSO₂CF₃. Vorzugsweise steht E in (I) für Brom, 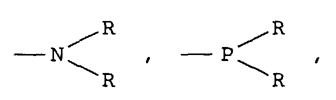 -OR, -SR und insbesondere für 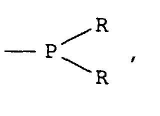 -OR, -SR oder Brom.

Die Reste R können gleich oder verschieden sein, vorzugsweise sind sie gleich. R steht für Wasserstoff, C₁- bis C₂₀-kohlenstofforganischer Rest oder C₁- bis C₃₀-siliciumorganischer Rest wie Trimethylsilyl, Triphenylsilyl.

Generell ist die chemische Konstitution der Reste R nicht kritisch. Bevorzugte C₁- bis C₂₀-kohlenstofforganische Reste R sind C₁- bis C₁₀-Alkyl oder C₃- bis C₁₀-Cyclolakyl, wie Methyl, Ethyl, n-Propyl, i-Propyl, n-Butyl, sec.-Butyl, i-Butyl, tert-Butyl, n-Pentyl, n-Hexyl, n-Octyl, n-Decyl,Cyclopropyl, Cyclobutyl, Cyclopentyl und Cyclohexyl. Bevorzugte derartige Reste sind Methyl, Ethyl, Cyclohexyl insbesondere Ethyl. Sehr gut geeignete Reste R sind ferner C₆- bis C₂₀-Arylreste, C₇-C₂₀-Arylalkyl oder C₇- bis C₂₀-Alkylarylreste, mit 6 bis 15 Kohlenstoffatomen im Arylrest. Die Arylreste können substituiert sein, beispielsweise mit C₁- bis C₁₀-Alkylresten oder Halogenatomen wie Chlor, Brom, Iod oder Fluor, oder aber mit anderen Arylresten so daß Biphenylreste resultieren, die auch "ortho-ortho" mit dem Heteroatom verbunden sein können. Beispielsweise seien genannt Phenyl, Benzyl, para-, ortho-, meta-Xylyl, para-, meta-, ortho-Tolyl oder auch Mesityl, ortho-, meta-, para-Chlorphenyl, ortho-, meta-, para-Trifluormethylphenyl.

Besonders bevorzugte aromatische oder aromatsubstituierte Reste R sind Phenyl, meta-Xylyl, 2,2'-Biphenyldiyl, Benzyl.

Als besonders bevorzugte Substituenten E sind zu nennen Diphenylphosphin -P(C₆H₅)₂, Di(metaxylyl)phosphin, 5-Dibenzophospholyl 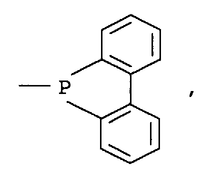 Diethylphosphin -P(C₂H₅)₂, Thiobenzyl -S(CH₂C₆H₅).

Die Reste R¹, R², R³, R⁴, R⁵, R⁶ sind gleich oder verschieden und haben die bereits für R definierte Bedeutung, vorzugsweise sind R¹, R², R³, R⁴, R⁵, R⁶ Wasserstoff, Methyl, Ethyl, Phenyl, insbesondere Wasserstoff.

Der Substituent Z in (I) steht für eine C₅- bis C₅₀-Cyclopentadienyl-Struktureinheit. Hierunter ist der Cyclopentadienylrest selbst C₅H₅, sowie alle einkernigen und mehrkernigen substituierten oder unsubstituierten Molekülstrukturen mit insgesamt 5 bis 50 Kohlenstoffatomen zu verstehen, in welchen die Cyclopentadienylstruktureinheit formal enthalten ist. Es seien beispielhaft genannt ein- bis vierfach mit C₁- bis C₂₀-kohlenstofforganischen oder C₁- bis C₃₀-siliciumorganischen Resten R substituierte Cyclopentadienderivate, wobei Definition und Vorzugsbereiche für R die bereits in (I) für R definierten sind. Beispielhaft seien genannt Methylcyclopentadienyl, tert-Butylcyclopentadienyl.

Als mehrkernige Derivate der Cyclopentadienylstruktureinheit seien Indenyl, Fluorenyl, Benzindenyl, sowie deren ein- bis achtfach mit C₁- bis C₂₀-kohlenstofforganischen oder C₁- bis C₃₀-siliciumorganischen Resten R substituierten Derivate, wobei die Definition und Vorzugsbereiche für R die gleichen wie die für R in (I) definierten sind, genannt.

Bevorzugte Substituenten Z in (I) sind Cyclopentadienyl 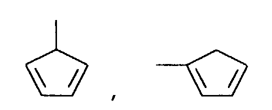 und andere Tautomere, Indenyl 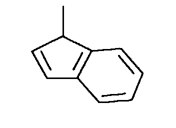 und andere Tautomere, Fluorenyl

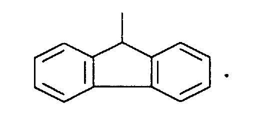

Die Bindung der Gruppe Z an den Rest des Molekuls der Formel (I) geschieht in der Regel über den Cyclopentadienyltyp-Fünfring der Gruppe Z, wobei aufgrund von Tautomerie in Z im allgemeinen ein Tautomerengemisch für (I) vorliegt.

T in (I) bedeutet Wasserstoff, C₁- bis C₂₀-kohlenstofforganischer Rest, C₁- bis C₃₀-siliciumorganischer Rest oder eine Gruppe E-Y-worin E 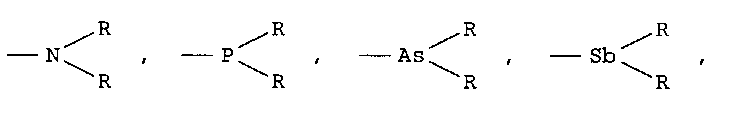 -OR, -SR, -SeR, -TeR oder eine Abgangsgruppe X bedeutet und wobei R die bereits für E in (I) definierte Bedeutung hat.

Y steht für eine C₁- bis C₂₀-organische Gruppe, die E mit C¹ in (I) verbindet.

Vorzugsweise steht Y für C₁- bis C₁₀-α-ω-Alkandiyleinheiten, wie 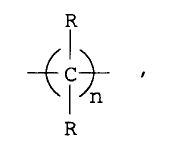 wobei R, gleich oder verschieden, die für R in (I) bereits definierte Bedeutung hat, aber insbesondere Wasserstoff bedeutet und n eine ganze Zahl von 1 bis 10 ist. Gut geeignete Struktureinheiten Y sind 1,2-Ethandiyl-CH₂-CH₂, 1,3-Propandiyl-CH₂-CH₂-CH₂-, insbesondere Methylen-CH₂-.

Eine weitere bevorzugte Struktureinheit Y wird durch folgende Strukturformel beschrieben:

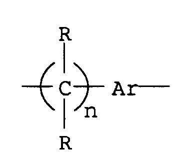

Hierin haben R, gleich oder verschieden, die in (I) bereits definierte Bedeutung, vorzugsweise sind beide R Wasserstoff, n ist vorzugsweise 1. Ar ist eine C₆- bis C₂₀-aromatische Struktureinheit, vorzugsweise C₆R₄, worin R die in (I) definierte Bedeutung hat und vorzugsweise Wasserstoff ist. Der Arylrest ist vorzugsweise paradisubstituiert. Eine gut geeignete Struktureinheit ist

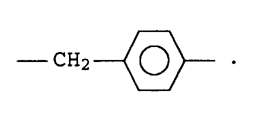

E in T ist vorzugsweise RO- oder eine Abgangsgruppe X, wobei R die bereits in (I) definierte Bedeutung hat, vorzugsweise Wasserstoff oder C₁- bis C₁₀-Alkyl, C₁- bis C₆-Aryl, Formyl, Acetyl, Propionyl bedeutet. Die Abgangsgruppe X ist vorzugsweise Fluor, Chlor, Iod und insbesondere Brom, der Methansulfonsäurerest CH₃-SO₂-, Trifluormethansulfonsäurerest CF₃SO₂- oder p-Toluolsulfonsäurerest p-(C₆H₄CH₃)-SO₂-.

Gut geeignete Gruppen T sind Methyl, Ethyl, Phenyl, Hydroxymethyl -CH₂-OH, Acetylmethyl -CH₂-OC(O)CH₃, Chlormethyl -CH₂Cl, Brommethyl -CH₂Br, Mesylmethyl -CH₂-OSO₂CH₃.

Tripodale Cyclopentadiene der Formel (I), in welchen alle, an das Kohlenstoffatom C¹ gebundenen, Gruppen verschieden sind, können als racemische Gemische, enantiomerenrein oder als Diastereomeren, letzteres in der Regel wenn die an C¹ gebundenen Gruppen bereits ein Chiralitätszentrum haben, vorliegen.

Geeignete Herstellungsverfahren für die erfindungsgemäßen tripodalen Cyclopentadiene (I) gehen im allgemeinen von den Oxetanderivaten der allgemeinen Formel (II) aus:

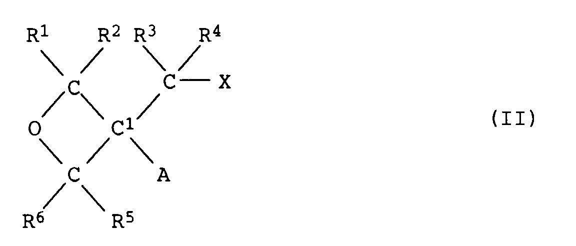

Hierin haben die Substituenten R¹, R², R³, R⁴, R⁵, R⁶ und die bereits in (I) definierte Bedeutung. X ist eine Abgangsgruppe im Sinne der Definition aus [a) Th. H. Lowry, K. Schueller Richardson, Mechanismen und Theorie in der Organischen Chemie, Verlag Chemie, Weinheim, 1980, S. 165, b) J. March, Advanced Organic Chemistry, 3th ed., John Wiley & Sons, New York, 1985, S. 179]. Generell sind alle Gruppen X als gute Abgangsgruppen geeignet, deren konjugierte Säure HX eine starke Säure ist.

Derartige Säuren HX sind beispielsweise Fluorwasserstoff-, Chlorwasserstoff-, Iodwasserstoff und vorzugsweise Bromwasserstoffsäure, weiterhin organische Sulfonsäuren wie Methylsulfonsäure CH₃-SO₃H, p-Toluolsulfonsäure p-CH₃-C₆H₄-SO₃H, Trifluormethansäure CF₃SO₃H. Die Säuren können in Substanz oder aber in, vorzugsweise, wäßriger Lösung eingesetzt werden. Im allgemeinen werden die Halogenwasserstoffsäuren in wäßriger Lösung eingesetzt.

Gut geeignete Abgangsgruppen X sind somit die Substituenten Fluor, Chlor, Iod und insbesondere Brom, weiterhin organische Sulfonsäurederivate wie die Methylsulfonyl-CH₃-SO₃-, p-Toluolsulfonyl- p-C₆H₄-SO₃-, Trifluormethansulfonylgruppe CF₃-SO₃-Benzolsulfonyl-C₆H₅-SO₃.

A in (II) bedeutet Wasserstoff, C₁- bis C₂₀-kohlenstofforganischer oder C₁- bis C₃₀-siliciumorganischer Rest R, mit der Definition von R in (I), oder eine Struktureinheit -Y-X, worin Y eine C₁- bis C₂₀-organische Gruppe ist, die X mit C¹ verbindet. X ist dann eine Abgangsgruppe, wie bereits definiert, vorzugsweise Brom oder Methansulfonyl, p-Toluolsulfonyl und Y hat die bereits für (I) definierte Bedeutung.

Die Oxetanderivate (II) sind nach [a) D. B. Pattison, J. Am. Chem. Soc., 1957, 79, 3455-3456, b) J. Cheynd, P. Chabrier, J. Seyden-Penne, A. Habert-Somug, T. Strazalko, Bull. Soc. Chim., Fr. 1965, 694-700; G. Huttner et al., Z. Naturforsch. 1995, 50b, 1045] erhältlich. Sie können über verschiedene Reaktionswege, hier als Weg 1 und Weg 2 bezeichnet, zu den erfindungsgemäßen tripodalen Cyclopentadienderivaten (I) umgesetzt werden.

Weg 1 wird im allgemeinen dann gewählt, wenn der Substituent A in (II) praktisch inert ist, das heißt keine Gruppen enthält, die der nucleophilen Substitution zugänglich sind. Weg 1 wird daher bevorzugt gewählt, wenn A in (II) Wasserstoff, C₁- bis C₂₀-kohlenstofforganischer oder C₁- bis C₃₀-siliciumorganischer Rest bedeutet; beispielhaft seien als Substituent A genannt, Wasserstoff, Methyl, Ethyl, n-Propyl, i-Propyl, n-Butyl, sec-Butyl, tert.-Butyl, i-Butyl, Cyclohexyl, Benzyl, Phenyl, Trimethylsilyl, vorzugsweise Methyl.

Die inert substituierten Oxetanderivate (II) werden dann via Weg 1 mit einem Cyclopentadienidanionenäquivalent oder einem substituierten Cyclopentadienidanionenäquivalent umgesetzt. Diese erhält man im allgemeinen, in dem man das zugrunde liegende Cyclopentadien C₅H₆ oder dessen Derivate Z-H, worin Z die bereits für (I) definierte Bedeutung hat, mit den bekannten Methoden, beispielsweise durch Umsetzung mit Metallalkylverbindungen wie Butyllithium oder Ethylmagnesiumchlorid in die entsprechenden metallierten Cyclopentadienidderivate (Z)ₙMet Halₘ umsetzt wobei Met Lithium, Natrium, Kalium, Rubidium, Cäsium, Beryllium, Magnesium, Calcium, Strontium, Barium, Bor, Aluminium, Gallium, Indium oder Thallium bedeutet. Vorzugsweise bedeutet Met Lithium, Magnesium und Hal bedeutet Fluor, Brom, Iod und insbesondere Chlor. n ist 1,2 oder 3, m 0, 1, 2 und m + n ist in Summe die formale Wertigkeit von Met in (Z)ₙMetHalₘ.

Die Umsetzung von (II) mit dem Cyclopentadienidanionenäquivalent wird im allgemeinen in organischen Lösungsmitteln wie Alkanen, Aromaten und vorzugsweise Ethern wie Diethylether, Tetrahydrofuran, Dioxan oder deren Mischungen bei einer Temperatur im Bereich von 50°C bis 100°C durchgeführt. In der Regel wird dann das Reaktionsgemisch hydrolysiert. Das Reaktionsgemisch kann direkt weiterverwendet, oder aber zum Reinprodukt (IIa) aufgearbeitet werden, worin R¹ bis R⁶, Z die für (I) definierte Bedeutung haben und A vorzugsweise inert ist und in (II) definiert ist.

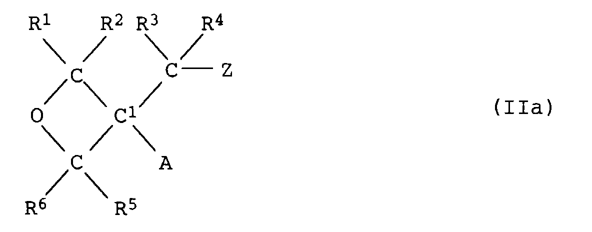

(IIa) kann jetzt auf zwei unterschiedlichen Wegen zu den tripodalen Cyclopentadienderivaten umgesetzt werden.

Bei Weg 1a) setzt man im allgemeinen (IIa) mit einer Säure H-X, die bereits definiert wurde, um und öffnet den Oxetanring zu den Verbindungen (IIba) oder (IIbb), oder deren Mischungen, je nach Regioselektivität der Reaktion

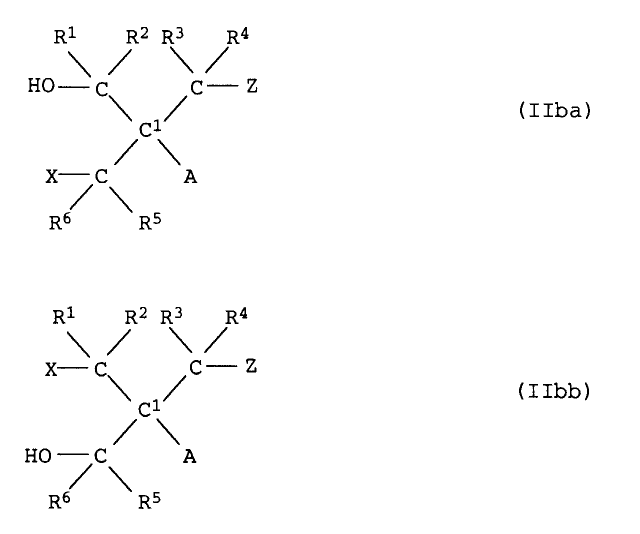

Hierin haben die Substituenten R¹ bis R⁶, Z und X die bereits in (I) und der Substituent A die bereits in (II) definierten Bedeutungen.

Die Umsetzung (IIa) zu (IIba) oder (IIbb) wird im allgemeinen in aliphatischen, aromatischen oder etherischen organischen Lösungsmitteln, beispielsweise Hexan, Toluol, Ether, Tetrahydrofuran oder deren Mischungen im Temperaturbereich von -100 bis 100°C, vorzugsweise -100 bis 0°C durchgeführt. Ein besonders bevorzugtes Reagenz H-X, ist wäßrige Bromwasserstoffsäure. Das erhaltene Produktgemisch wird üblicherweise noch wäßrig-basisch aufgearbeitet und kann direkt weiterverarbeitet werden oder aber mit üblichen Methoden der präparativen organischen Chemie gereinigt werden.

Die Verbindungen (IIba) oder (IIbb) stellen wertvolle Zwischenprodukte dar, da sie einerseits selbst schon als Ligand fungieren können, andererseits aber noch breit derivatisiert werden können.

Hiernach wird die OH-Funktion in (IIba) oder (IIbb) mit den üblichen Methoden der organischen Chemie in eine Abgangsgruppe X umgewandelt. Vorzugsweise setzt man hierzu (IIba) oder (IIbb) mit Sulfonsäurederivaten wie Methansulfonsäurechlorid, Trifluormethansulfonsäurechlorid oder p-Toluolsulfonsäurechlorid um und erhält (IIc) worin X gleich oder verschieden sein können, je nach der Natur der in der Synthesesequenz verwendeten Reagenzien zur Einführung der Abgangsgruppe X und die Reste R¹ bis R⁶, Z, X die in (I) und der Rest A die in (II) definierte Bedeutung hat.

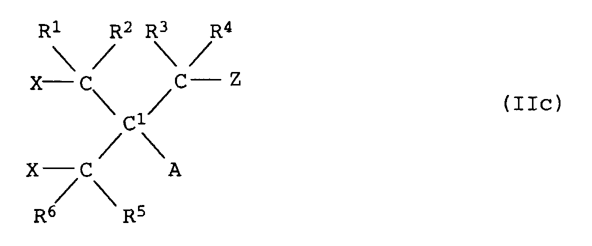

Ein alternativer Syntheseweg zu racemischen oder enantiomerenreinen Verbindungen der Formel (IIc) geht von einer racemischen oder enantiomerenreinen Vorstufe von IIc aus, in welcher Z durch eine Abgangsgruppe X, wie definiert, substituiert ist und alle X verschieden sind (IIc-1). Eine derartige, bevorzugte Verbindung ist racemisches oder enantiomerenreines CH₃C(CH₂Br) (CH₂Cl)CCH₂OSO₂CF₃) das, beispielsweise nach G. Huttner et al., Chem. Ber. 1994, S. 271-274 und Z. Naturforsch. 1995, 50b, S. 729-734, erhältlich ist. Die Umsetzung von (IIc-1) mit einem bereits definierten Cyclopentadienidanionenäquivalent (Z)ₙMetHalₘ, vorzugsweise C₅H₅Li, C₅H₅K, führt dann zu einer Verbindung (IIc), welche als Racemat oder enantiomerenrein vorliegen kann.

Die Umsetzung von (IIc) mit Met(E)ₙ, wobei Met Lithium, Natrium, Kalium, Rubidium, Cäsium, Calcium, Magnesium, Beryllium, Barium, Strontium, vorzugsweise jedoch Lithium oder Kalium bedeutet, n die maximale Wertigkeit von Met in Met(E)ₙ ist und 1 oder 2 bedeutet und E die bereits für (I) definierte Bedeutung, außer X hat, führt zu den tripodalen Cyclopentadienderivaten (I) in welchen dann T vorzugsweise für Wasserstoff, C₁- bis C₂₀-kohlenstofforganischen oder C₁- bis C₃₀-siliciumorganischen Rest, wie in Formel (I) spezifiziert, steht.

Die vorgenannten Umsetzungen werden üblicherweise in aliphatischen, aromatischen organischen oder vorzugsweise etherischen Lösungsmitteln durchgeführt, gut geeignete Lösungsmittel sind Tetrahydrofuran, Diethylether, Toluol. Die Umsetzungstermperaturen liegen üblicherweise im Bereich von -100 bis 100°C, vorzugsweise im Bereich von -30 bis 80°C.

Das molare Verhältnis Met(E)ₙ:(IIc) liegt im allgemeinen im Bereich um 10:1 bis 2:1, vorzugsweise 5:1 bis 2:1 und insbesondere bei 3,5:1.

Als Alternative zu dem beschriebenen ersten Syntheseweg 1a) hat sich eine Syntheseroute 1b) erwiesen, bei welcher die nucleophile Öffnung des Oxetanderivats (IIa) eine Rolle spielt.

Hierzu setzt man im allgemeinen das bereits definierte Oxetanderivat (IIa) mit dem bereits definierten Reagenz Met(E)ₙ zu den Verbindungen (IIda) oder (IIdb), oder zu einer Mischung aus (IIda) und (IIdb) um.

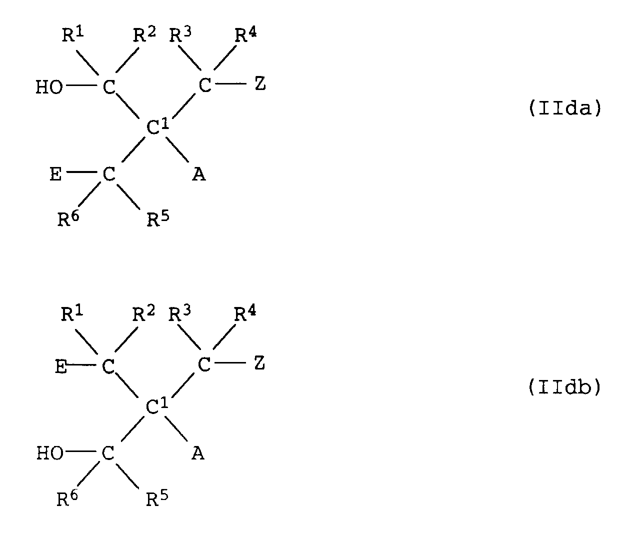

Sind alle Substituenten des zentralen Kohlenstoffatoms C¹ in (IIda) (IIdb) zudem unterschiedlich, so treten im allgemeinen Stereoisomere, beispielsweise Enantiomere, von (IIda) oder (IIdb) auf. Die Reste R¹ bis R⁶, Z, E und A in (IIda) (IIdb) haben die bereits für (I) oder (IIa) definierte Bedeutung.

Die Verbindungen (IIda), (IIdb) stellen wertvolle Zwischenprodukte dar, da sie einerseits bereits als Liganden in Metallkomplexen verwendet werden können und andererseits, insbesondere an der OH-Funktion, noch chemisch breit variierbar sind.

Im allgemeinen wird die OH-Funktion in (IIda), (IIdb) mit den üblichen Methoden der organischen Chemie in einen Abgangsgruppe X, die bereits definiert wurde, umgewandelt.

Vorzugsweise setzt man hierzu (IIda) oder (IIdb) mit Sulfonsäurederivaten wie Methansulfonsäurechlorid, Trifluormethansulfonsäurechlorid, p-Toluolsulfonsäurechlorid, Benzolsulfonsäurechlorid oder auch wäßrigem Bromwasserstoff um und erhält (IIea) oder (IIeb), die wiederum Regioisomere und Stereoisomere darstellten können. Die Reste R¹ bis R⁶, Z, E und A haben die bereits für (I) oder (IIa) definierte Bedeutung.

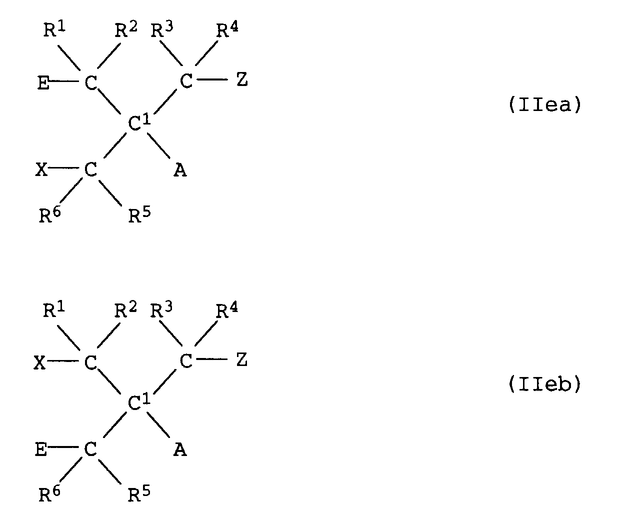

Als sehr gut geeignete Variante zur Überführung der OH-Funktion in eine Abgangsgruppe X hat sich die folgende Sequenz herausgestellt.

Das durch die Umsetzung von (IIa) zu (IIda), (IIdb) erhältliche rohe Produktgemisch wird im allgemeinen mit Boran-Komplexen, beispielsweise Boran-Tetrahydrofuranaddukt BH₃·(OC₄H₈), umgesetzt, anschließend kann die OH-Funktion, wie bereits beschrieben, in die Abgangsgruppe X übergeführt werden, beispielsweise durch Umsetzung des vorliegenden Rohprodukts mit Methansulfonylsäurechlorid/Triethylamin; anschließend kann das Boran mit einer starken Lewisbase, beispielsweise einem Amin wie Morpholin, entfernt werden, und man erhält (IIea) oder (IIeb).

Die Umsetzung von (IIea), (IIeb) mit Met(E)ₙ, wobei Met Lithium, Natrium, Kalium, Rubidium, Cäsium, Calcium, Magnesium, Beryllium, Barium oder Strontium, vorzugsweise Lithium oder Kalium bedeuten, n die maximale Wertigkeit von Met in Met(E)ₙ ist und 1 oder 2 bedeutet und E die bereits für (I) definierte Bedeutung, vorzugsweise -P(R) (R) , hat, führt dann unter Substitution von X zu den erfindungsgemäßen tripodalen Cyclopentadienderivaten (I) worin T vorzugsweise für Wasserstoff, C₁- bis C₂₀-kohlenstofforganischer oder C₁- bis C₃₀-siliciumorganischer Rest, wie in Formel (I) spezifiziert, steht.

Ein Vorteil der Syntheseroute Weg 1b ist, daß sie sehr gut geeignet ist unterschiedliche Reste E in das Molekül (I) einzuführen um somit gegebenenfalls zur Stereoisomeren oder gar reinen Enantiomeren von (I) zu gelangen.

Syntheseweg 2 wird im allgemeinen gewählt, um funktionalisierte tripodale Cyclopentadienderivate (III) herzustellen.

Der Substituent A in (II) enthält im allgemeinen eine Gruppe, die der nucleophilen Substitution zugänglich ist. Gut geeignete Gruppen A sind solche mit den Strukturen -Y-X, worin Y für eine C₁- bis C₂₀-organische Gruppe steht, die X mit C¹ in (II) verbindet und bereits definiert wurde. Vorzugsweise steht Y für C₁- bis C₁₀-α-ω-Alkandiyleinheiten, wie (̵C(R) (R))̵ₙ, wobei R gleich oder verschieden ist und die für R bereits definierte Bedeutung hat, aber insbesondere Wasserstoff bedeutet und n eine Zahl von 1 bis 10, vorzugsweise 1 bis 3 ist. Geeignete Y sind 1,2-Ethandiyl -CH₂-CH₂-, 1,3-Propandiyl -CH₂-CH₂-CH₂- und insbesondere Methylen -CH₂-.

Y kann auch für eine C₆- bis C₂₀-aromatische Gruppe stehen, wie p-Phenylen, p-Biphenylen, p-Xylylen und vorzugsweise para-CH₂-C₆H₄-.

X steht für die bereits definierten Abgangsgruppen, vorzugsweise für Brom, Methansulfonyl CH₃SO₂-O-.

Verbindung (II), mit dem für A definierten -Y-X-Substituent, wird dann üblicherweise mit den bereits definierten Cyclopentadienylanionenäquivalent MetₙZXₘ und den bereits definierten Reagenz Met (E)ₙ, vorzugsweise Met P(R) (R), worin Met vorzugsweise Lithium oder Kalium und R vorzugsweise Phenyl, Ethyl oder m-Xylyl bedeutet, zum Oxetanderivat umgesetzt, welches wieder, je nach Substitution in R¹ bis R⁶ und Y, in Form verschiedener Isomerer vorliegen kann (IIIaa), (IIIab).

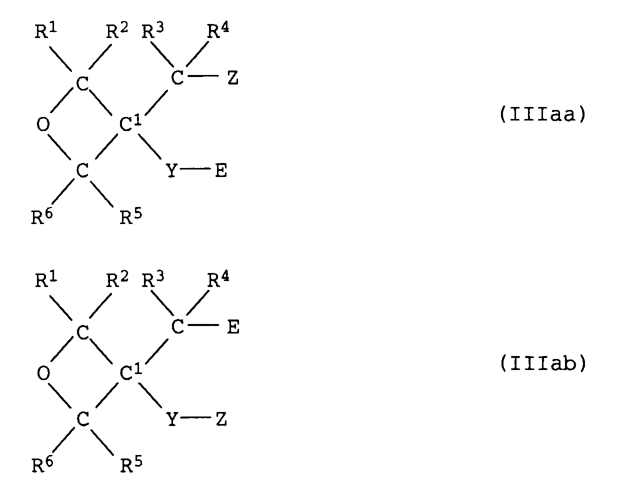

Um die Isomerenvielfalt zu reduzieren setzt man üblicherweise symmetrische Oxetanderivate (II) ein, also beispielsweise solche wo Y identisch mit -C(R³) (R⁴)- ist; ganz besonders bevorzugt sind solche, wo Y und -C(R³) (R⁴)- in (II) die Methylengruppe -CH₂- bedeuten.

Die gegebenenfalls isomeren Verbindungen (IIIaa), (IIIab) werden dann im allgemeinen mit dem Reagenz Met(E)ₙ, vorzugsweise ist E -P(R) (R) mit R Phenyl, Ethyl, m-Xylyl, umgesetzt und der Oxetanring zu den Verbindungen (IIIba), (IIIbb), (IIIbc), (IIIbd) nucleophil geöffnet.

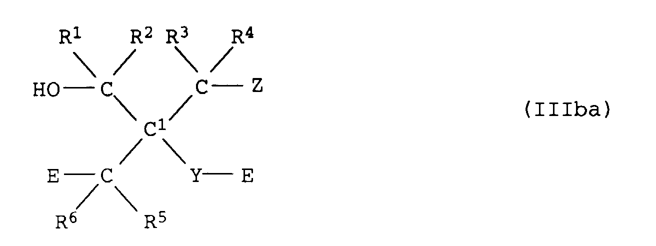

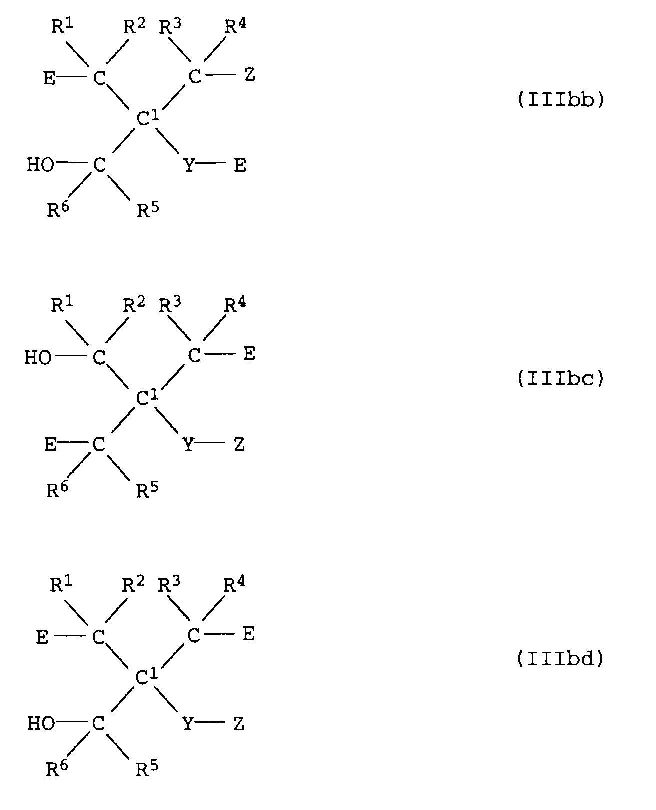

Hier wird im allgemeinen, um die Isomerenvielfalt zu verringern, der Bedingung genügt, daß alle Einheiten -C(R¹) (R²)-, -C(R³) (R⁴)-, -C(R⁵) (R⁶)- und -Y- identisch sind und insbesondere für -CH₂- stehen; vorzugsweise bedeutet E dann -P(R) (R).

Die Umsetzung der OH-Funktion in IIIba, IIIbb, IIIbc oder IIIbd mit Silylierungs-, Alkylierungs- oder Acylierungsreagentien R-X oder 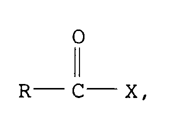 worin R die in (I) definierte Bedeutung hat und X eine Abgangsgruppe wie bereits für (I) definiert bedeutet, kann dann zu den Verbindungen (IIIca), (IIIcb), (IIIcc) oder (IIIcd) führen, worin R' RO oder

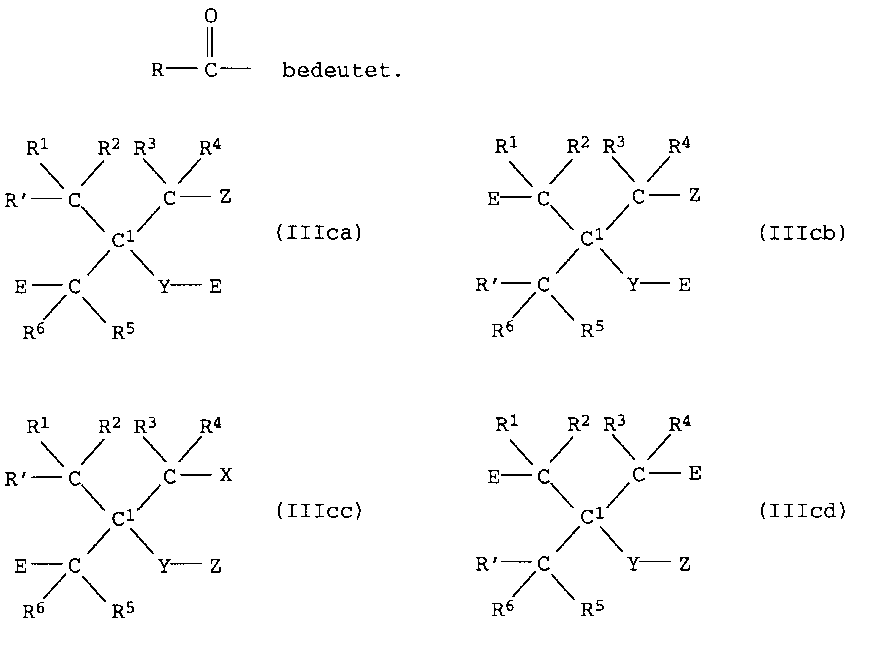

Die Verbindungen (IIIca) bis (IIIcd) insbesondere jene, in welcher die Reste die bereits definierte Bedeutung haben und wo R¹ bis R⁶ vorzugsweise Wasserstoff, Y vorzugsweise Wasserstoff, E vorzugsweise für -P(R) (R) und R' , vorzugsweise für Acyl R-C(O)- steht, wobei R die bereits definierte Bedeutung, vorzugsweise -CH₃ hat, sind wertvolle tripodale, funktionalisierte Liganden, deren Sauerstoffunktionalität R' beispielsweise als Ankergruppe für die chemische oder physikalische Anbindung an Trägermaterial, wie anorganische Oxide, also Kieselgel SiO₂, Aluminiumoxid, Al₂O₃ oder Polymere, wie funktionalisierte Polystyrole, beispielsweise Merrifield Polymer, dienen kann.

Die Reaktionsbedingungen für die vorgenannten Umsetzungen des Synthesewegs 2 sind im allgemeinen nicht kritisch.

Die Umsetzungen werden im allgemeinen bei Temperaturen im Bereich von -50°C bis 150°C, üblicherweise in einem organischen Lösungsmittel, vorzugsweise Alkohole, aromatische Kohlenwasserstoffe und insbesondere Tetrahydrofuran, Toluol, Pyridin durchgeführt. Die Verbindungen der Synthesesequenz II nach III können entweder direkt als Rohprodukt weiterverarbeitet werden, oder aber zunächst isoliert und gereinigt werden.

Die folgenden Schemata 1 bis 8 sollen die Variationsbreite der unterschiedlichen Synthesewege und die Variationsbreite der Verbindungen des Typs (I) illustrieren.

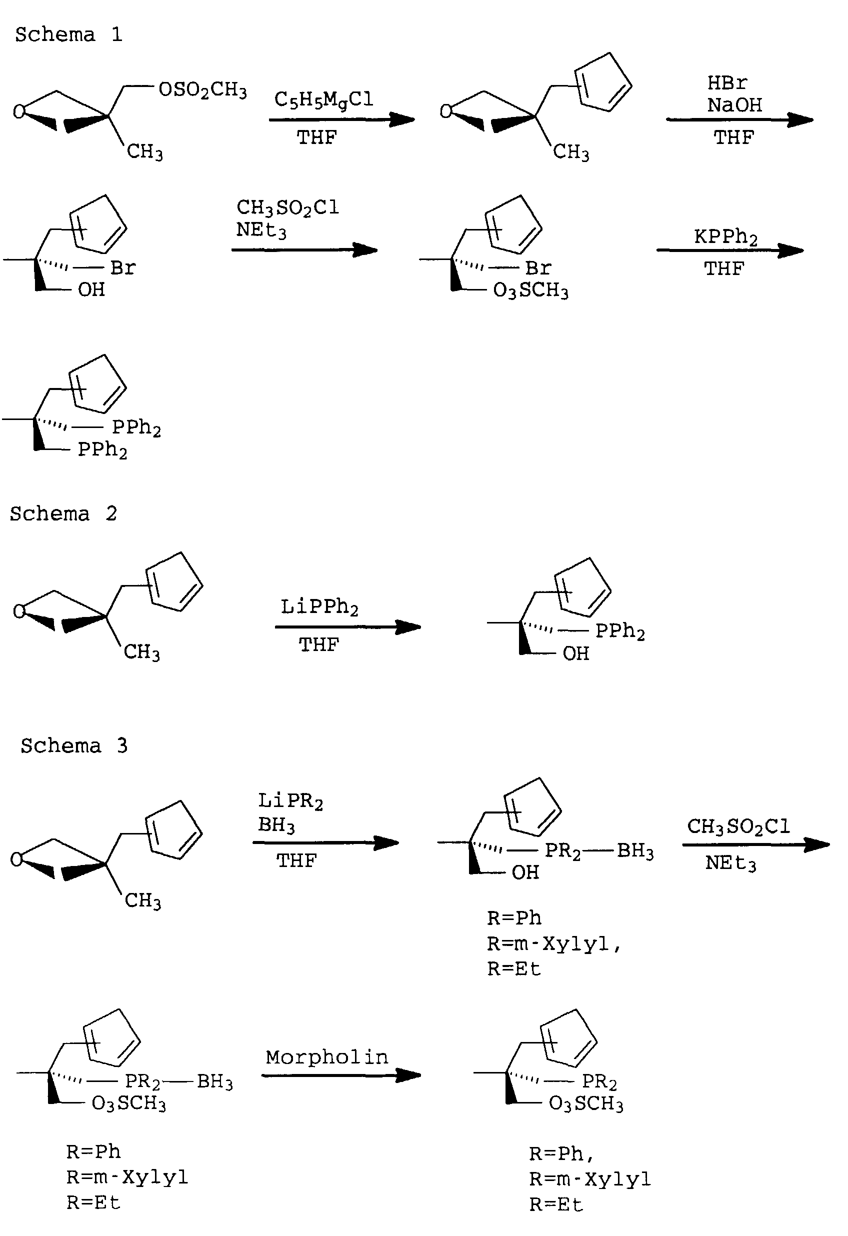

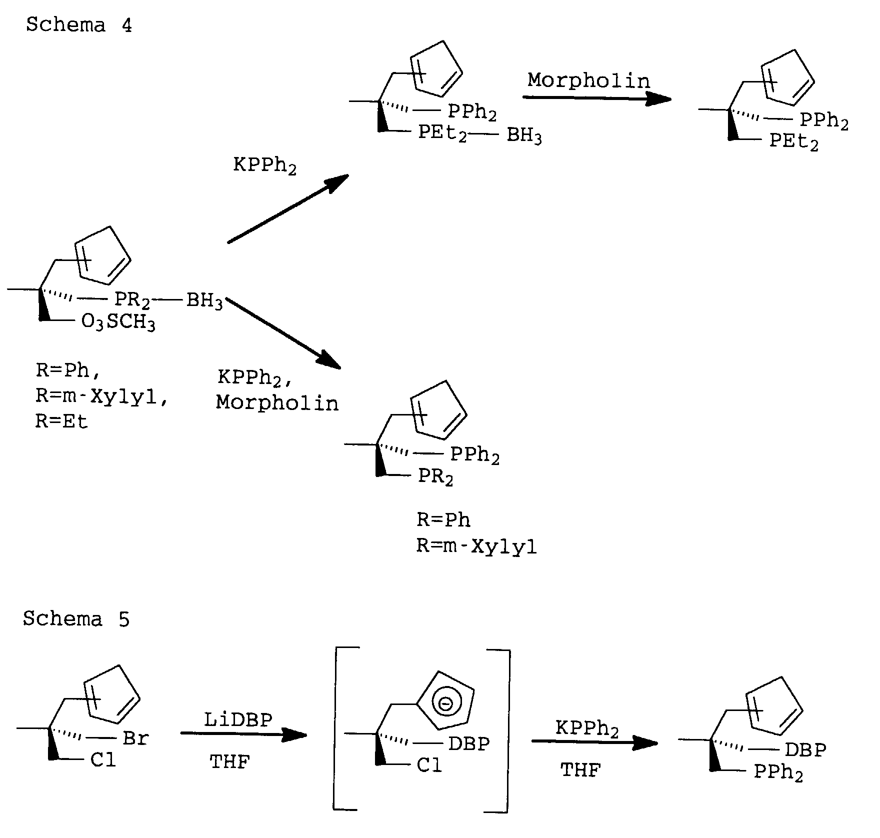

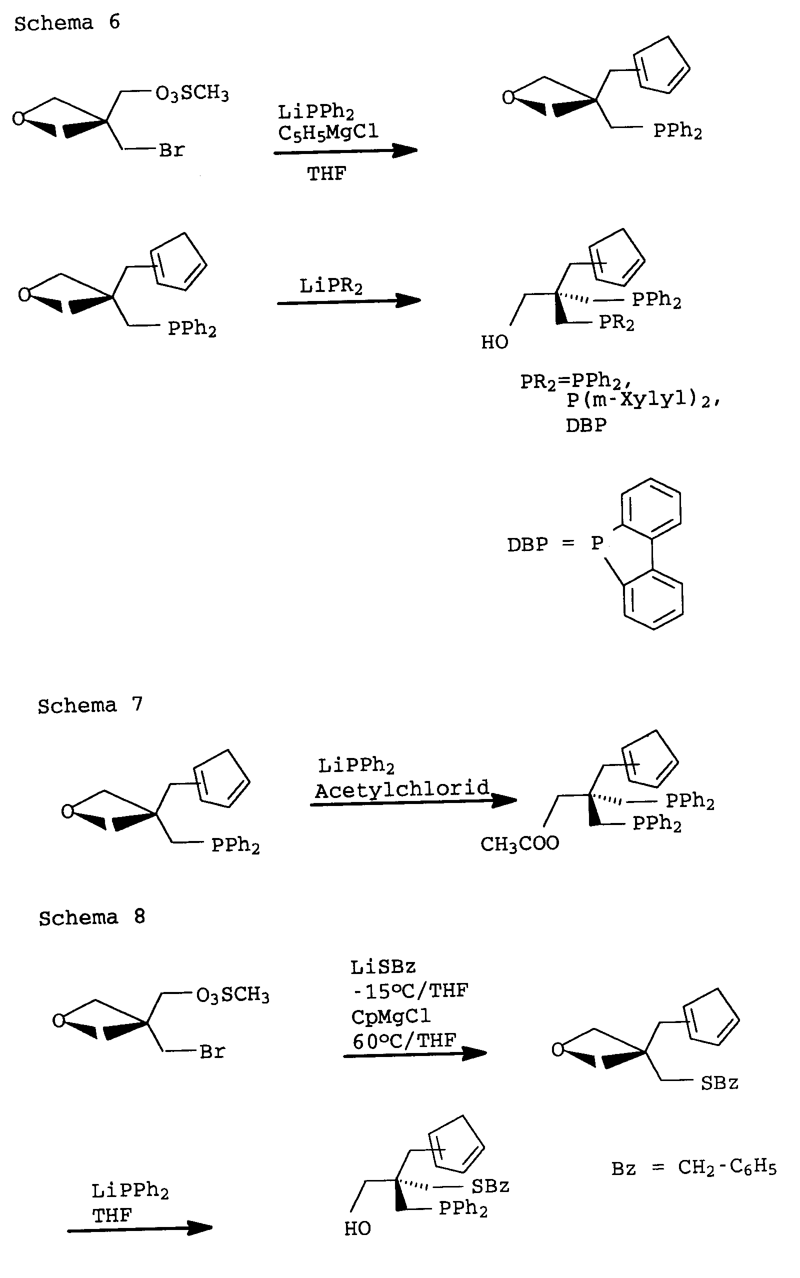

Besonders bevorzugte Verbindungen der allgemeinen Formel (I) sind beispielsweise: 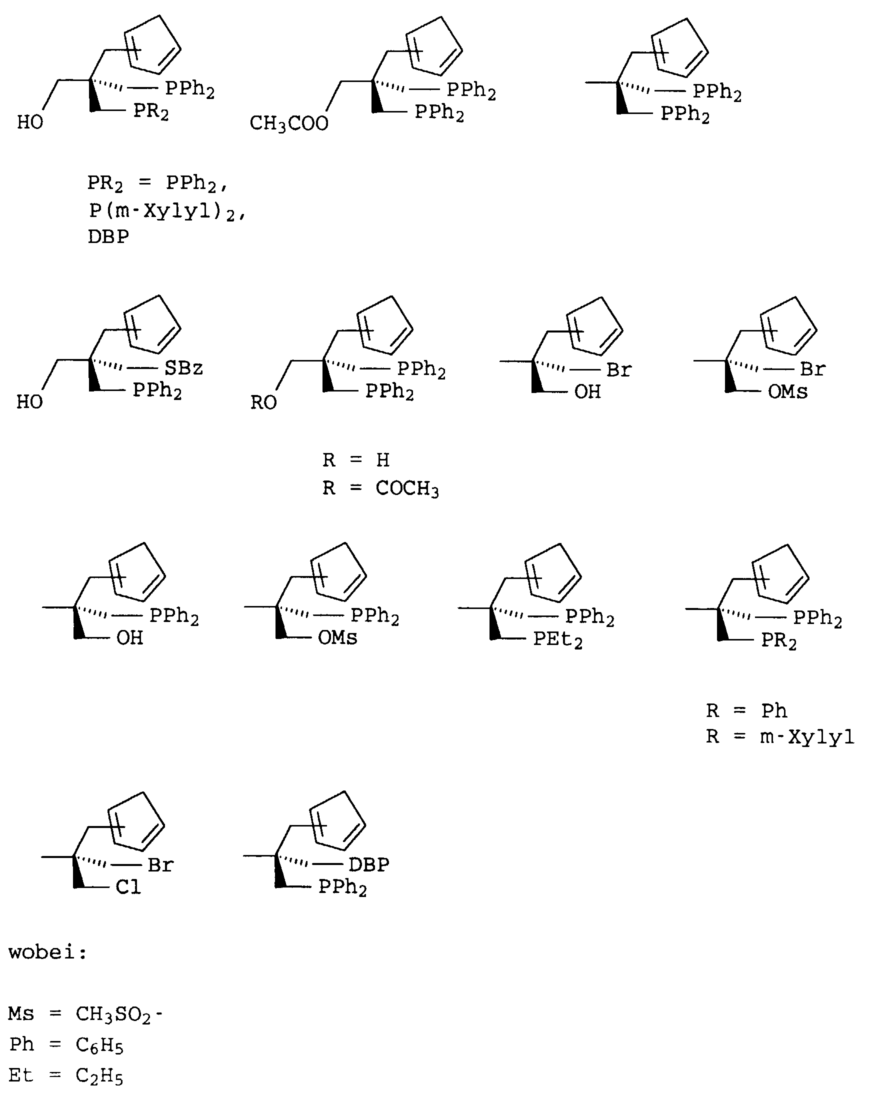 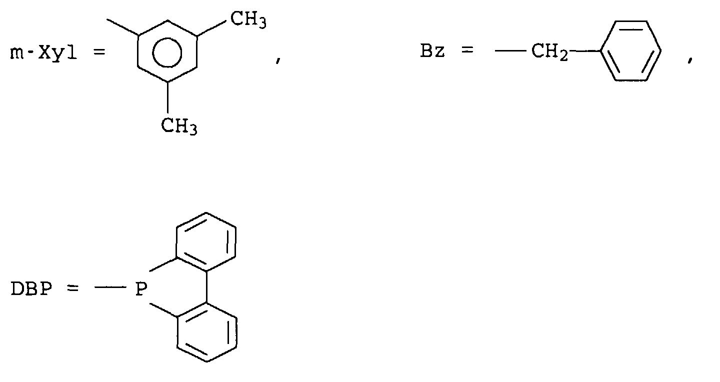 bedeuten.

Die erfindungsgemäßen tripodalen Cyclopentadienderivate (I) sind gut geeignet als Liganden in Tripodmetallkomplexen LₙM(Tp)ₘ (V), worin M ein Übergangsmetall oder Hauptgruppenmetall des Periodensystems der Elemente, vorzugsweise ein Metall aus der Gruppe der Übergangsmetalle des Periodensystems und insbesondere Ti, Zr, Hf, Fe, Mn, Mo, Ru, Co ist, L ein anionischer oder neutraler Ligand, wie Kohlenmonoxid, C₁- bis C₂₀-organischer Rest, wobei die chemische Natur von L nicht kritisch ist und Tp die bereits definierten tripodalen Cyclopentadienderivate (I), welche einfach deprotoniert sein können, und im allgemeinen über die Cyclopentadienylstruktureinheit, vorzugsweise pi-artig und insbesondere η⁵-an das Zentralmetall M gebunden sind. n ist eine ganze Zahl von 0 bis 7, m ist eine ganze Zahl von 1 bis 8.

Die Herstellung der Metallkomplexe (V) erfolgt im allgemeinen durch Umsetzung eines tripodalen Cyclopentadienderivats (I) oder eines einfach deprotonienten tripodalen Cyclopentadienderivats (I') mit einer Metallverbindung, welche sübstitutionsfähige Reste trägt, wie für analoge Umsetzungen mit nichtfunktionalisierten Cyclopentadieniden beispielsweise in J. Organomet. Chem. 1989, 369, S. 359-370 beschrieben.

Die deprotonienten tripodalen Cyclopentadienderivate (I') erhält man üblicherweise durch Umsetzung von (I) mit starken, im allgemeinen metallorganischen, Basen der ersten, zweiten oder dritten Hauptgruppe des Periodensystems der Elemente wie C₁-C₆-Alkylverbindungen des Lithiums, Natriums, Kaliums, Cäsiums, Magnesiums, vorzugsweise verwendet man als Base n-Butyllithium, Alkali-Erdalkalihydroxide, Alkali- oder Erdalkalialkoholate wie Natriummethacrylat, NaOEt, KOtBu; Amide wie Lithiumdiisopropylamid.

Üblicherwiese wird ein H-Atom aus der Cyclopentadienylstruktureinheit entfernt und formal gegen das Metall der metallorganischen Base ausgetauscht.

Derartige Deprotonierungs- oder Metallierungsreaktionen sind dem Fachmann bekannt.

Als substitutionsfähige Reste der Metallverbindung kommen generell jene in Frage, deren Komplexbindungsstärke mit dem in Frage kommenden Metall M geringer ist, als die Komplexbindungsstärke des verdrängenden tripodalen Cyclopentadienylliganden (I) oder Cyclopentadienidliganden (I').

Gut geeignete substitutionsfähige Reste sind die Halogene, also Fluor, Chlor, Iod und insbesondere Brom, andere eingangs definierten Abgangsgruppen X, Hydrid, ferner Kohlenmonoxid, Mono-Olefine wie Ethylen, Propen, Cyclohexen, Cyclopenten, Norbornen, Diolefine, wie 1,3-Butadien, Cyclooctadien, Ether wie Tetrahydrofuran, Diethylether; Amine NR₃ wie Triethylamin, Phosphine PR₃ wie PPh₃, PMe₃, Nitrile wie Acetonitril, β-Diketoverbindungen wie Acetylaceton.

Geeignete Metallverbindungen, die substitutionsfähige Reste tragen sind beispielsweise alle Metallhalogenide, insbesondere die Chloride und Bromide, Metallcarbonylhalogenide beispielsweise Pentacarbonylmangan(I)bromid, (CO)₅MnBr, Eisen(II)chlorid, Fe(CO)₄Br, Metallcarbonylnitrile.

Üblicherweise wird das tripodale Cyclopentadienderivat (I) oder (I') in einem organischen Lösungsmittel, vorzugsweise Tetrahydrofuran, vorgelegt und die Metallverbindung, vorzugsweise eine Halogenmetallverbindung, zugegeben und gerührt. Die Reaktionstemperatur liegt im allgemeinen im Bereich von -100 bis 150°C, vorzugsweise im Bereich von -80 bis 30°C.

Die Tripodkomplexe (V) lassen sich mannigfaltig chemisch modifizieren, beispielsweise ergibt die Umsetzung von Tripodmetallhalogenid-Komplexen mit Salzen von nicht-nucleophilen Anionen, beispielsweise Natriumhexafluorophosphat, Natriumtetrafluoroborat, Lithiumtetrakis(pentafluorophenyl)borat die kationischen Metallkomplexe mit den nicht-nucleophilen Anionen als Gegenion.

Eine weitere bevorzugte Modifizierung ist der Austausch der Halogenliganden in Tripodmetallhalogenidkomplexen beispielsweise gegen organische Reste R oder Wasserstoff, wobei R die in (I) definierte Bedeutung haben kann, beispielsweise Phenyl, Methyl. Üblicherweise wird ein Tripodmetallhalogenidkomplex bei niedriger Temperatur, vorzugsweise im Bereich von -100 bis 30°C mit Organometallverbindungen Met R, wobei Met ein Metall aus der ersten, zweiten oder dritten Hauptgruppe des Periodensystems ist und R die in (I) definierte Bedeutung hat umgesetzt. Beispielhaft seien für Met R Phenyllithium, n-Butyllithium, Methyllithium oder Grignard-Verbindungen, wie Phenylmagnesiumchlorid, Methylmagnesiumchlorid genannt.

Die tripodalen Liganden Tp sind im allgemeinen sowohl über ihre Cyclopentadienidstruktureinheit als auch über mindestens eines ihrer E-Donorzentren an das Metallatom M gebunden. Eine bevorzugte Bindungsweise ist, wenn sowohl die Cyclopeltandienylstruktureinheit (η⁵) als auch zwei Donorzentren E, wobei E vorzugsweise -P(R) (R) ist, wie bereits definiert, an das Metallatom M gebunden sind (Tripodgeometrie).

Die erfindungsgemäßen Liganden (I) können vorteilhaft für die Herstellung von Metallkomplexen verwendet werden. Die Metallkomplexe können als Mediatoren oder als Katalysatoren oder als Bestandteil von Katalysatoren oder Mediatoren für stereo- oder regioselektive Umsetzungen in der organischen Chemie, die stöchiometrische oder katalytische Kohlenstoff-Kohlenstoff-Bindungsknüpfung oder Hydrierung, beispielsweise die Hydrierung von C=C- oder C=O-Doppelbindungen, verwendet werden.

Weiterhin eignen sich die Metallkomplexe selbst als Katalysatoren, oder als Bestandteil von katalytisch aktiven Mischungen, insbesondere für die Polymerisation von Kohlenwasserstoffmonomeren oder funktionalisierten Monomeren. Beispielhaft seien genannt C=C-Monomere wie, Alk-1-enen, also Ethen, Propen, 1-Buten, 1-Hexen, 1-Octen oder Acrylsäure oder deren Derivate wie Methylacrylat, Ethylacrylat, n-Butylacrylat oder Styrol und dessen Derivate.

### Beispiele

### Allgemeines

Sämtliche Arbeiten wurden unter getrocknetem Argon in Schlenk-Gefäßen durchgeführt.

Die folgenden Chemikalien wurden gemäß Literatur hergestellt:
3-Methansulfonoxymethyl-3-methyl-3-oxetan (**1**) nach J. Cheymol, P. Chabrier, J. Seyden-Penne, A. Habert-Somny, T. Strazalko, *Bull*. *Soc*. *Chim*. *Fr*. **1965**, 694;
Trifluormethansulfonsäure-3-brom-2-chlormethyl-1-propylester (**11**) nach H. Heidel, G. Huttner, G. Helmchen, *Z*. *Naturforsch*., *Teil B* **1993**, *48*, 1681;
(CO)₅MnBr nach W. P. Fehlhammer, W. A. Herrmann, K. Öfele in Brauer, *Handbuch der Präparativen Anorganischen Chemie*, *Vol*. *3*, Ferdinand Enke Verlag, Stuttgart 1978;
1,1-Bis(diphenylphosphanomethyl)-1-cyclopentadienylmethylethan (**5a**), 1-Cyclopentadienyl-2-(di-m-xylylphosphanomethyl)-2-(diphenylphosphanomethyl)-propan (**5b**) und 1-Cyclopentadienyl-2-diethylphosphanomethyl-2-diphenylphosphanomethylpropan (**5c**) wurden wie in den Beispielen 5, 15, 17 beschrieben hergestellt.
Diphenylphosphan und Di-m-xylylphosphan nach R. E. Ireland, D. M. Walba, *Org*. *Synth*. **1977**, *56*, 47;
5-H-Dibenzophosphol nach J. Cornforth, R. H. Cornforth, R. T. Grey, *J*. *Chem*. *Soc*. *Perkin Trans*. *I*. **1982**, 2289 bzw. H. Braye, I. Caplier, R. Saussez, *Tetrahedron* **1971**, *27*, 5523;
(Cyclopentadienyl-magnesiumchlorid nach J. R. Stille, R. H. Grubbs, *J*. *Org*. *Chem*. **1989,** *54*, 434.
Mo(CH₃CN)₃(CO)₃ nach D. B. Tate, W. R. Knipple, J. M. Augl, *Inorg*. *Chem*. **1962**, *7*, 433
(PPh₃)₂FeCl₂: L. H. Pegnolet, D. Forster, W.d.W. Horrocks, Inorg. Chem., 1968, 7, S. 828

### Analytik:

UV/VIS-Spektrophotometer Lambda 9 der Fa. Perkin Elmer, CH₂Cl₂-Lösungen in Küvetten vom Typ Hellma 110 suprasil (0.2 cm Schichtdicke)
Cyclovoltammetrie: Meßbedingungen: 10⁻³ M Lösung in 0.1 M n-Bu₄NPF₆/CH₃CN-Lösung in einer Metrohm-Zelle, Spanungsvorschub 200 mV/sec, Potentiale in Volt gegen gesättigte Kalomel-Elektrode an Glassy-Carbon-Elektrode bei 25°C, Potentiostat / Galvanostat Model 273 der Fa. EG & G Princeton Applied Research.
ESR-Spektren wurden mit einem Bruker ESP 300E Spektrometer aufgenommen (X-Band, externer Standard DPPH (Diphenylpikrylhydrazyl)).
Photochemische Reaktionen: Durchführung in einer kühlbaren Duranglas-50-Apparatur mit einer Hanau TQ 150 Quecksilberhochdrucklampe.
NMR: Bruker AC-200 (298 K) (¹H: 200 MHz; ¹³C: 50 MHz). Interner Standard durch Lösungsmittel CDCl₃ (d = 7.27 für ¹H, 77.0 für ¹³C) relativ zu TMS extern. ³¹P: 81 MHz, Standard H₃PO₄ (85%) extern. Die ¹³C- und ³¹P-NMR-Spektren wurden ¹H-entkoppelt aufgenommen. Abkürzungen: bs (breites Signal), d (Dublett), dd (Dublett von Dubletts), t (Triplett), pt (Pseudotriplett), m (Multiplett), Singuletts sind nicht gesondert ausgezeichnet. Alle Messungen erfolgten in CDCl₃ als Lösungsmittel.
MS: Finnigan MAT 8230 mit Datensystem SS 300, EI (70 eV), FAB (Matrix: 4-Nitrobenzylalkohol, Triethanolamin); die *m*/*z*-Werte beziehen sich auf das jeweils häufigste Isotop.
Elementaranalysen: Mikroanalytisches Laboratorium des Organisch-Chemischen Instituts der Universität Heidelberg.

Die numerierten Strukturformeln sind im Anschluß an die Beispiele zusammengestellt.

### Präparatives:

### Beispiel 1: 3-Cyclopentadienylmethyl-3-methyl-oxetan (2)

In einem 500 ml Dreihalskolben mit Tropftrichter, Rückflußkühler und Schutzgasanschluß wurden 70.0 g (230 mmol) CpMgCl*2THF unter Luft- und Feuchtigkeitsausschluß in 200 ml THF gelöst. Die dunkle Lösung wurde auf 60°C erhitzt und bei dieser Temperatur eine Lösung von 28.8 g (192 mmol) **1** in 100 ml THF über 2h zugetropft. Anschließend ließ man die Lösung 2 h unter Rückfluß kochen. Nach Abkühlen wurde mit 200 ml 10%-Ammoniumchloridlösung hydrolysiert. Die wäßrige Phase wurde dreimal mit 50 ml Diethylether extrahiert und die vereinigten organischen Phasen bis zur Neutralität mit gesättigter Kochsalzlösung gewaschen. Nach Trocknen über Natriumsulfat wurde das gelbe Öl im Vakuum fraktioniert destilliert. **2** geht bei 63-66°C (2,7 mbar) als farbloses bis leicht gelbliches Öl über, Ausb. 18.1 g (63%). MS (EI); *m*/*z* (%) [Frag.]: 150 (10%) [M⁺], 105 (100%) [M⁺-CH₂O-CH₃], 79 (70%) [HC₅H₄CH₂⁺]. C₁₀H₁₄O (150.22): ber.: C 79.97, H 9.39, O 10.64; gef: C 79.22, H 9.44.

### Beispiel 2: 2-Brommethyl-2-cyclopentadienylmethyl-1-propanol (3)

In einem 500 ml Schlenkrohr (8 cm Durchmesser) wurden 4.5 g (30 mmol) **2** in 150 ml THF gelöst und auf -70°C gekühlt. Die wäßrige HBr-Lösung wurde während 5 min zugetropft und die Lösung noch 2 h bei dieser Temperatur gerührt. Nach Entfernen des Kühlbads taute die Lösung innerhalb 1.5 h auf -15°C auf. Durch Zugabe von 40 ml 10%-Natronlauge wurde die Reaktionslösung bei dieser Temperatur hydrolysiert und eine Stunde ohne Kühlung gerührt. Die Lösung nahm während dieser Zeit Raumtemperatur an. Die wäßrige Phase wurde abgetrennt und dreimal mit 30 ml Diethylether ausgeschüttelt. Die vereinigten organischen Phasen wurden anschließend mit gesättigter Kochsalzlösung gewaschen und über Natriumsulfat getrocknet. Nach Entfernen des Lösungsmittels erhielt man ein gelbes Öl. Das so erhaltene Rohprodukt (6,97 g) kann ohne weitere Reinigung weiter umgesetzt werden. Zur Charakterisierung wurde ein Teil des Rohprodukts an einer Kieselgelsäule blitzchromatographiert (4*25 cm; Eluens: PE/THF im Verhältnis 9 : 1; R_{F} = 0.18). Man erhielt nach Einengen der Produktfraktionen **3** als farbloses Öl.
MS (EI); *m*/*z* (%) [Frag.]: 231 (10 %) [M⁺], 79 (100 %) [HC₅H₄CH₂⁺]. C₁₀H₁₅BrO (231.132): ber. C 51.97, H 6.54, Br 34.57, O 6.92; gef. C 52.21, H 6.58, Br 34.55.

### Beispiel 3: Methansulfonsäure-2-brommethyl-2-cyclopentadienylmethyl-1-propylester (4)

In einem 200 ml Schlenkrohr mit Septum wurden 6.97 g Rohprodukt **3** und 4.35 ml (45 mmol) Triethylamin in 100 ml Methylenchlorid gelöst. Bei 0°C wurden 2.37 ml (31 mml) Methansulfonylchlorid innerhalb von 2 min zugespritzt. Nach Hälfte der Zugabe fiel ein farbloser Niederschlag aus. Die Suspension wurde noch eine halbe Stunde bei dieser Temperatur gehalten und dann 2 h bei Raumtemperatur gerührt. Die Reaktionslösung wurde anschließend mit 20 ml Wasser hydrolysiert. Die Phasen wurden getrennt und die wäßrige Phase noch dreimal mit 20 ml Methylenchlorid extrahiert. Die vereinigten organischen Phasen wurden mit Kochsalzlösung bis zur Neutralität gewaschen und über Natriumsulfat getrocknet. Entfernen des Lösungsmittels im Ölpumpenvakuum ergab ein leicht bräunliches Öl, das in Methylenchlorid gelöst und auf Kieselgur aufgezogen wurde. Es folgt Blitzchromatographie an einer Kieselgelsäule (4*25cm; PE/THF im Verhältnis 8:2; R_{F} = 0.42). Nach Einengen der Produktfraktionen im Ölpumpenvakuum erhielt man 5.9 g (66%) **4** als farbloses Öl.
MS (EI); *m*/*z* (%) [Frag.]: 308 (7%) [M⁺-1], 133 (30%) [M⁺-HBr-OMs], 79 (100%) [HC₅H₄CH₂⁺]. C₁₁H₁₇BrO₃S (309.218): ber. C 42.73, H 5.54, Br 25.84O 15.52, S 10.37; gef. C 42.62, H 5.58.

### Beispiel 4: Arbeitsvorschrift zur Herstellung von Kaliumdiphenylphosphid

Zu einer etwa 0.3 M Lösung von Diphenylphosphan in THF wurde bei 0°C ein Äquivalent KOtBu zugegeben und die entstandene leuchtend rote Lösung mindestens eine halbe Stunde nachgerührt.

### Beispiel 5: 1,1-Bis(diphenylphosphanomethyl)-1-cyclopentadienylmethyl-ethan (5a)

Methode A: In einem 500 ml Dreihalskolben mit Septum, Rückflußkühler und Inertgasanschluß wurden 3 Äquivalente einer Kaliumdiphenylphosphidlösung vorgelegt und eine Lösung von 6.18 g (20 mmol) **4** in 70 ml THF innerhalb von 2 min zugegeben. Die Lösung wurde 3 h unter Rückfluß erhitzt und nach Abkühlen das Lösungsmittel im Ölpumpenvakuum entfernt. Der Rückstand wurde in 70 ml Diethylether aufgenommen und mit 30 ml Wasser hydrolysiert. Die wäßrige Phase wurde abgetrennt und dreimal mit 30 ml Diethylether extrahiert. Die vereinigten organischen Phasen wurden mit Koschsalzlösung zur pH-Neutralität gewaschen und über Natriumsulfat getrocknet. Im Ölpumpenvakuum wurde das Lösungsmittel abgezogen, der erhaltene zähe, farblose Rückstand in Methylenchlorid gelöst und auf Kieselgur aufgezogen. Blitzchromatographie an einer Kieselgelsäule (12 * 8 cm; Eluens: PE/Diethylether im Verhältnis 9.75 : 0.25; R_{F} = 0.32) ergab nach Entfernen der Lösungsmittels **5a** als ein farbloses Öl, Ausb. 6.0 g (60 %).

### Beispiel 6: Allgemeine Arbeitsvorschrift zur Herstellung von Lithiumdibenzophospholid, Lithiumdiaryl- und Lithiumdialkylphosphiden

Zu einer etwa 0.3 M Lösung des Diaryl- bzw. Dialkylphosphans in THF wurde bei 0°C in etwa 5 min ein Äquivalent n-BuLi-Lösung in Hexan (ca. 2.3 M) getropft. Anschließend ließ man die Lösung noch mindestens eine halbe Stunde nachrühren. Die so hergestellten roten Lösungen wurden unmittelbar zu Synthese eingesetzt.

### Beispiel 7: 2-Cyclopentadienylmethyl-2-diphenylphosphanomethyl-1-propanol (6)

In einem 250 ml Dreihalskolben mit Rückflußkühler, Septum und Inertgasanschluß wurden 1.4 g (13.4 mmol) **2** in 50 ml THF gelöst und bei 0°C durch Zuspritzen von 5.6 ml (13.4 mmol) n-BuLi-Lösung deprotoniert. Es wurde eine halbe Stunde nachgerührt. Innerhalb einer halben Stunde wurden dazu bei Raumtemperatur über einen Kapillarschlauch 1.5 Äquivalente einer Lithiumdiphenylphosphidlösung zugetropft. Die Reaktionslösung wurde anschließend zwei Stunden unter Rückfluß gekocht. Die Reaktionsmischung wurde im Ölpumpenvakuum weitgehend vom Lösungsmittel befreit und der Rückstand mit 50 ml Diethylether aufgenommen. Zur Hydrolyse gab man 20 ml Wasser zu und rührte 10 min nach. Die organische Phase wurde abgetrennt und die wäßrige Phase zweimal mit 30 ml Diethylether extrahiert. Die vereinigten Etherphasen wurden mit Kochsalzlösung zur pH-Neutralität gewaschen und über Natriumsulfat getrocknet. Das verbleibende farblose Öl wurde in Methylenchlorid gelöst und auf Kieselgur aufgezogen. Es folgte Blitzchromatographie an Kieselgel (20 * 4 cm; Eluens: PE/THF im Verhältnis 8.5: 1.5; R_{F} = 0.30). Man erhielt **6** nach Entfernen des Lösungsmittels als farbloses, zähes Öl, Ausb. 3.37 g (75%).
MS (EI); *m*/*z* (%) [Frag.]: 337 (100%) [M⁺+1], 183 (24%) [PPh₂⁺-2H]. C₂₂H₂₅OP (336.413): ber. C 78.54, H 7.49, O 4.76, P 9.21; gef. C 77.94, H 7.42.

### Beispiel 8: 2-Cyclopentadienylmethyl-2-diphenylphosphanomethylboran-1-propanol (7a)

Die Durchführung und Aufarbeitung zum Rohprodukt erfolgte analog Beispiel 7 aus einem Ansatz von 1.55 g (10.32 mmol) **2** in 50 ml THF und 1.2 Äquivalenten einer Diphenylphosphidlösung.

Nach Aufarbeitung der Reaktionslösung wurde das Rohprodukt **6** in 20 ml THF gelöst und auf 0°C im Eisbad abgekühlt. Innerhalb einer halben Minute wurden 14.9 ml einer 1M Lösung von BH₃ in THF zugespritzt. Man ließ eine halbe Stunde bei dieser Temperatur nachrühren und entfernt dann das Lösungsmittel im Ölpumpenvakuum. Das verbleibende farblose Öl wurde in Methylenchlorid gelöst und auf Kieselgur aufgezogen. Es folgt Blitzchromatographie an Kieselgel (20 * 4 cm; Eluens: PE/THF im Verhältnis 7.35: 2.65, RF = 0.30). Nach Entfernen des Lösungsmittels erhielt man **7a** als ein farbloses, zähes Öl, Ausb. 2.42 g (67%).
MS (FAB); *m*/*z* (%) [Frag.]: 349 (100%) [M⁺-1]. C₂₂H₂₈BOP (350.257): ber. C 75.44, H 8.06, B 3.09, O 4.57, O 4.57, P 8.84; gef. C 74.97, H 8.10, P 8.57.

### Beispiel 9: 2-Cyclopentadienylmethyl-2-di-m-xylylphosphanomethylboran-1-propanol (7b)

Die Darstellung erfolgte analog der von **7a**. Der Ansatz, 2.28 g (15.2 mmol) **2**, deprotoniert mit 7 ml (15.2 mmol) n-BuLi-Lösung und 1.2 Äquivalenten Lithiumdi-m-xylylphosphid lieferte das Rohprodukt von **7b**, das mit 21.9 ml einer 1M Lösung von BH₃ in THF umgesetzt wurde. Man erhielt nach Blitzchromatographie an Kieselgel (20 * 4 cm; Eluens: PE/THF im Verhältnis 8.5: 1.5; R_{F} = 0.28) 4.9 g (80%) **7b** als ein farbloses, zähes Öl.
MS (FAB); *m*/*z* (%) [Frag.]: 405 (45%) [M⁺-1], 241 (100%) [P(m-Xyl)₂⁺]. C₂₆H₃₆OPB (406.364): ber. C 76.84, H 8.87, B 2.71, O 3.94, P 7.64; gef. C 75.47, H 9.19.

### Beispiel 10: 2-Cyclopentadienylmethyl-2-diethylphosphanomethylboran-1-propanol (7c)

Die Darstellung erfolgte analog der von **7a**. Der Ansatz, 1.5 g (10 mmol) **2**, deprotoniert mit 4 ml (10 mmol) n-BuLi-Lösung und 1.2 Äquivalenten Lithiumdiethylphosphid lieferte das Rohprodukt von **7c**, das mit 15.6 ml einer 1M Lösung von BH₃ in THF umgesetzt wurde. Man erhielt nach Blitzchromatographie an Kieselgel (20 * 4cm; Eluens: PE/THF im Verhältnis 7.5:2.5; R_{F} = 0.39) 2.0 g (78%) **7c** als ein farbloses, zähes Öl.
MS (EI); *m*/*z* (%) [Frag.]: 254 (38%) [M⁺], 240 (25%) [M⁺-BH₃], 225 (15%) [M⁺-C₂H₅], 209 (16%) [M⁺-CH₂OH-BH₃], 117 (60%) [M⁺-BH₃-CH₂PEt₂]. C₁₄H₂₈BOP (254.168): ber. C 66.16, H 11.10, B 4.25, O 6.30, P 12.19; gef. C 66.08, H 11.04, P 12.17.

### Beispiel 11: Methansulfonsäure-2-cyclopentadienylmethyl-2-diphenylphosphanomethyl-boran-1-propylester (8a)

In einem 250 ml Dreihalskolben mit Inertgasanschluß wurden 2.4 g (6.86 mmol) **7a** in 50 ml Methylenchlorid gelöst und mit 1.4 ml (10.3 mmol) Triethylamin versetzt. Bei 0°C spritzte man 0.7 ml (8.91 mmol) Methansulfonylchlorid zu und ließ die Reaktionslösung 16 h bei Raumtemperatur rühren. Nach Hydrolyse mit 20 ml Wasser wurde die organische Phase abgetrennt und die wäßrige Phase dreimal mit je 50 ml Methylenchlorid extrahiert. Die vereinigten organischen Phasen wurden anschließend mit gesättigter Kochsalzlösung bis zur Neutralität gewaschen und über Natriumsulfat getrocknet. Einengen der Lösung im Ölpumpenvakuum ergab ein bräunliches Öl, welches erneut in Methylenchlorid gelöst und auf Kieselgur aufgezogen wurde. Nach Blitzchromatographie an Kieselgel (20 * 4 cm; Eluens: PE/THF im Verhältnis 7: 3; R_{F} = 0.30) erhielt man **8a** in Form eines farblosen Feststoffs, Ausb. 2.2 g (75%).
MS (EI); *m*/*z* (%) [Frag.]: 428 (10%) [M⁺], 414 (50%) [M⁺-BH₃], 335 (35%) [M⁺-BH₃-SO₂CH₃], 183 (100%) [PPh₂⁺-2H]. C₂₃H₃₀BO₃PS (428.339): ber. C 64.49, H 7.06, B 2.52, O 11.21, P 7.24, S 7.48; gef. C 64.52, H 7.21, P 7.98.

### Beispiel 12: Methansulfonsäure-2-cyclopentadienylmethyl-2-di-m-xylylphosphanomethyl-boran-1-propylester (8b)

Die Darstellung erfolgte analog der von **8a**. Der Ansatz, 4.46 (11.46 mmol) **7b** mit 2.4 ml (17.35 mmol) Triethylamin und 1.2 ml (14.9 mmol) Methansulfonylchlorid in 70 ml Methylenchlorid, lieferte nach Blitzchromatographie an Kieselgel (20 * 4 cm; Eluens: PE/THF im Verhältnis 8:2; R_{F} = 0.31) **8b** als farblosen Feststoff, Ausb. 3.7 g (67%).
MS (FAB); *m*/*z* (%) [Frag.]: 484 (3%) [M⁺], 470 (40%) [M⁺-BH₃], 241 (100%) [P(m-Xyl)₂⁺]. C₂₇H₃₈BOPS (484.447): ber. C 66.94, H 7.91, B 2.23, O 9.91, P 6.39, S 6.62; gef. C 64.02, H 7.60.

### Beispiel 13: Methansulfonsäure-2-cyclopentadienylmethyl-2-diethylphosphanomethyl-boran-1-propylester (8c)

Die Darstellung erfolgte analog der von **8a**. Der Ansatz, 3.42 g (13.4 mmol) **7c** mit 3 ml (21.4 mmol) Triethylamin und 1.3 ml (16 mmol) Methansulfonylchlorid in 70 ml Methylenchlorid, lieferte nach Blitzchromatographie an Kieselgel (25 * 4 cm; Eluens: PE/THF im Verhältnis 7.5:2.5; R_{F} = 0.38) **8c** als farblosen Feststoff, Ausb. 3.6 g (82%).
MS (EI); *m*/*z* (%) [Frag.]: 332 (11%) [M⁺], 318 (50%) [M⁺-BH₃], 253 (80%) [M⁺-SO₂CH₃]; 223 (65%) [M⁺-CH₂OSO₂CH₃], 79 (75%) [HC₅H₄CH₂⁺ u. SO₂CH₃⁺]. C₁₅H₃₀BO₃PS (332.261): ber. C 54.19, H 9.10, B 3.31, O 14.45, P 9.32, S 9.63; gef. C 53.42, H 8.92.

### Beispiel 14: Methansulfonsäure-2-cyclopentadienylmethyl-2-diphenylphosphanomethyl-1-propylester (9)

Die Durchführung und Aufarbeitung zum Rohprodukt erfolgte analog der von **8a** aus einem Ansatz von 2.89 g (8.26 mmol) **7a**, 1.7 ml (12.39 mmol) Triethylamin und 0.85 ml (10.74 mmol) Methansulfonylchlorid in 50 ml Methylenchlorid.

Das aufgearbeitete Rohprodukt **8a** wurde in 2 ml Morpholin gelöst und 15 min bei 70°C gerührt. Nach Entfernen des Morpholins im Ölpumpenvakuum wurde das Öl in Methylenchlorid aufgenommen und auf Kieselgur aufgezogen. Anschließend folgte blitzchromatographische Reinigung an Kieselgel (20 * 4 cm; Eluens: PE/THF im Verhältnis 8: 2; R_{F} = 0.27). Einengen der Produktfraktionen im Vakuum ergaben **9** in Form eines farblosen Öls, Ausb. 2.5 g (73%).
MS (EI); *m*/*z* (%) [Frag.]: 414 (60%) [M⁺], 335 (50%) [M⁺-SO₂CH₃], 199 (55%) [CH₂PPh₂⁺], 183 (100%) [PPh₂⁺-2H]. C₂₃H₂₇O₃PS (414.507): ber. C 66.64, H 6.57, O 11.59, P 7.48, S 7.72; gef. C 66.23, H 6.74.

### Beispiel 15: 1-Cyclopentadienyl-2-(di-m-xylylphosphanomethyl)-2-(diphenylphosphanomethyl)-propan (5b)

In einem 250 ml Dreihalskolben mit Septum, Rückflußkühler und Inertgasanschluß wurden 3.5 Äquivalente der Kaliumdiphenylphosphidlösung vorgelegt und eine Lösung von 2.62 g (5.42 mmol) **9** in 50 ml THF innerhalb von 2 min zugegeben. Anschließend wurde die Reaktionsmischung 16 h bei Raumtemperatur gerührt. Dabei fiel ein farbloser Niederschlag aus. Die Reaktionsmischung wurde im Ölpumpenvakuum weitgehend vom Lösungsmittel befreit und der Rückstand mit 50 ml Diethylether aufgenommen. Zur Hydrolyse gab man 20 ml Wasser zu und rührte 10 min. Die organische Phase wurde abgetrennt und die wäßrige Phase zweimal mit 30 ml Diethylether extrahiert. Die vereinigten Etherphasen wurden mit Kochsalzlösung zur pH-Neutralität gewaschen und über Natriumsulfat getrocknet. Das verbleibende farblose Öl wurde in 5 ml Morpholin aufgenommen und eine halbe Stunde bei 70°C im Ölbad erhitzt. Das Morpholin wurde im Ölpumpenvakuum abgezogen, der Rückstand in Methylenchlorid gelöst und auf Kieselgur aufgezogen. Es folgte Blitzchromatographie an Kieselgel (20 * 4 cm; Eluens: PE/Diethylether im Verhältnis 9.75: 0.25; R_{F} = 0.31). Man erhielt 1.42 g (47%) **5b** als ein farbloses, zähes Öl.
MS (EI); *m*/*z* (%) [Frag.]: 560 (70%) [M⁺], 483 (100%) [M⁺-C₆H₅], 375 (98) [M⁺-PPh₂], 319 (50%) [M⁺-P(m-Xyl)₂], 241 (100%) [P(m-Xyl)₂⁺], 183 (85%) [PPh₂⁺-2H]. C₃₈H₄₂P₂ (560.699): ber. C 81.40, H 7.55, P 11.05; gef. C 80.43, H 7.63.

### Beispiel 16: 1-Cyclopentadienyl-2-diethylphosphanomethylboran-2-diphenylphosphanomethyl-propan (10)

Die Durchführung und Aufarbeitung zum Rohprodukt von **10** erfolgte analog **5b**, ohne daß dieses in Morpholin weiter umgesetzt wurde. Der Ansatz, 2.77 g (8.3 mmol) **8c** und 3 Äquivalente Kaliumdiphenylphosphid, lieferte nach Blitzchromatographie an Kieselgel (25 * 4 cm; Eluens: PE/Diethylether im Verhältnis 9.5: 0.5; R_{F} = 0.35). Einengen der Produktfraktionen im Ölpumpenvakuum ergaben 1.69 g (48%) **10** als ein farbloses, zähes Öl.
MS (EI); *m*/*z* (%) [Frag.]: 422 (9%) [M⁺], 408 (16%) [M⁺-BH₃], 379 (100%) [M⁺-BH₃-C₂H₅], 183 (22%) [PPh₂⁺-2H]. C₂₆H₃₇BP₂ (422.346).

### Beispiel 17: 1-Cyclopentadienyl-2-diethylphosphanomethyl-2-diphenylphosphanomethyl-propan (5c)

1.69 g (4 mmol) **10** wurden in 5 ml Morpholin gelöst und 2 h bei 80°C gerührt. Nach Entfernen des nicht umgesetzten Morpholins im Ölpumpenvakuum wurde das erhaltene farblose Öl in Methylenchlorid aufgenommen, auf Kieselgur aufgezogen und an Kieselgel säulenchromatographiert (25 * 3 cm; Eluens: PE/Diethylether im Verhältnis 9: 1; R_{F} = 0.55). Nach Entfernen des Lösungsmittels erhielt man **5c** in Form eines farblosen Öls, Ausb. 1.35 g (84%).
MS (EI); *m*/*z* (%) [Frag.]: 408 (6%) [M⁺], 379 (100%) [M⁺-CH₂CH₃], 331 (4%) [M⁺-C₆H₅], 183 (19%) [PPh₂⁺-2H]. C₂₆H₃₄P₂ (408.503): ber. C 76.45, H 8.39, P 15.16; gef. C 75.36, H 8.48, P 14.94.

### Beispiel 18: 2-Brommethyl-2-chlormethyl-1-cyclopentadienyl-propan (12)

In einem 250 ml Dreihalskolben mit Inertgasanschluß und Tropftrichter wurden 8.63 g (28.39 mmol) CpMgCl*2.5THF in 100 ml THF gelöst. Über den Tropftrichter wurde eine Lösung von 2.60 g (7.80 mmol) **11** in 50 ml THF während einer Stunde zugetropft. Anschließend ließ man die braune Reaktionsmischung 16 h bei Raumtemperatur rühren. Hydrolysiert wurde durch Zugabe von 50 ml 10%-Ammoniumchlorid-Lösung und 10 min Rühren. Die wäßrige Phase wurde abgetrennt und dreimal mit 30 ml Diethylether extrahiert. Die vereinigten organischen Phasen wurden über Natriumsulfat getrocknet und das Lösungsmittel im Ölpumpenvakuum entfernt. Das erhaltene gelbe Öl wurde in Methylenchlorid aufgenommen und auf Kieselgur aufgezogen. Es folgt Blitzchromatographie an Kieselgur (10 * 4 cm; Eluens: PE, R_{F} = 0.35). Nach Entfernen des Lösungsmittels im Ölpumpenvakuum erhielt man 1.70 g (87%) **12** in Form eines farblosen bis leicht gelblichen Öls.
MS (EI); *m*/*z*: (%) [Frag.]: 250 (18%) [M⁺], 79 (100%) [HC₅H₄CH₂⁺]. C₁₀H₁₅BrCl (249.578): ber. C 48.13, H 5.64, Br 32.01, Cl 14.22; gef. C 48.46, H 5.70

### Beispiel 19: 1-Cyclopentadienyl-2-(5-dibenzophospholylmethyl)-2-(diphenylphosphanomethyl)-propan (5d)

In einem 250 ml Schlenkrohr mit Septum wurden 1.58 g (6.31 mmol) **12** in 50 ml THF gelöst, bei 0°C durch Zugabe von 2.75 ml n-BuLi-Lösung deprotoniert und eine halbe Stunde nachgerührt. Innerhalb einer halben Stunde wurde dazu bei 0°C über einen Kapillarschlauch ein Äquivalent einer Lithiumdibenzophospholidlösung zugetropft. Die gelb-orangefarbene Reaktionsmischung wurde noch 2 h bei dieser Temperatur gerührt und anschließend im Ölpumpenvakuum auf etwa 30 ml eingeengt. Bei Raumtemperatur wurde die Reaktionslösung nun innerhalb einer Minute zu 3.5 Äquivalenten einer Kaliumdiphenylphosphidlösung gegeben und die Mischung 16 h bei Raumtemperatur gerührt. Anschließend entfernte man im Ölpumpenvakuum weitgehend das Lösungsmittel und nahm den Rückstand in 50 ml Diethylether auf. Zur Hydrolyse gab man 20 ml Wasser zu und rührte 10 min. Die organische Phase wurde abgetrennt und die wäßrige Phase zweimal mit 30 ml Diethylether extrahiert. Die vereinigten Etherphasen wurden mit Kochsalzlösung zur pH-Neutralität gewaschen und über Natriumsulfat getrocknet. Das Lösungsmittel wurde im Ölpumpenvakuum entfernt, der Rückstand in Methylenchlorid gelöst und auf Kieselgur aufgezogen. Es folgte Blitzchromatographie an Kieselgel (20 * 4 cm; Eluens: PE/Diethylether im Verhältnis 9.75: 0.25; R_{F} = 0.31). Man erhielt 0.7 g (22%) **5d** als farbloses, zähes Öl.
MS (EI); *m*/*z* (%) [Frag.]: 502 (25%) [M⁺-H], 319 (60%) [M⁺-H-DBP], 183 (100%) [DBP⁺]. C₃₄H₃₂P₂ (502.574): ber. C 81.26, H 6.42, P 12.32; gef. C 82.79, H 7.17.

### Beispiel 20: Carbonyl-[2,2-bis(diphenylphosphanomethyl)- η⁵-cyclopentadienylpropyl]-Mangan(I) (13)

In einem 100 ml-Schlenkrohr wurden 0.45 g (0.9 mmol) **5a** in 30 ml THF gelöst, mit 0.4 ml 2.3 molarer n-BuLi-Lösung deprotoniert und eine halbe Stunde nachgerührt. Zu dieser Lösung wurden bei Raumtemperatur 0.25 g (0.91 mmol) BrMn(CO)₅ zugegeben. Die orangefarbene Reaktionsmischung ließ man 1 h bei dieser Temperatur rühren und kochte die Lösung noch 3 h unter Rückfluß. Das Lösungsmittel wurde vollständig im Ölpumpenvakuum entfernt, der Rückstand in 30 ml Diethylether aufgenommen und über 2 cm Kieselgur in einer G3-Umkehrfritte filtriert. Das Filtrat wurde erneut im Ölpumpenvakuum eingeengt. Der erhaltene orangefarbene Rückstand wurde dreimal mit 10 ml Petrolether gewaschen und in 100 ml THF aufgenommen. Die Lösung wurde nun bei 5°C bestrahlt. Nach sechs Stunden zeigt ein IR-Spektrum der Reaktionslösung nur noch eine Carbonylbande bei n = 1829 cm⁻¹. Im Ölpumpenvakuum wurde das Lösungsmittel entfernt und der Rückstand auf Kieselgel aufgezogen. Es folgt Blitzchromatographie an Kieselgel (14*2 cm; Eluens: PE/THF im Verhältnis 8.5: 1.5; R_{F} = 0.47). Das Produkt läuft als kräftig orangefarbene Bande. Man erhielt 132 mg (25%) **13** in Form eines orangefarbenen Pulvers. Orangefarbene Kristalle, die zur Röntgenstrukturanalyse geeignet waren, erhielt man durch Gasphasendiffusion von Petrolether in eine Toluol-Lösung von **13** bei 3°C innerhalb von 24 h.
1H-NMR: d = 1.55 (bs, 3H, CH₃); 1.88 (2H, CH₂Cp); 2.13 (m, 1H, CH₂ₐP, 2J_{HH} = 15.1 Hz, 2J_{HP} = 5 Hz, 4J_{Hp} = 4.1 Hz); 2.40 (m, 1H, CH_{2b}P, 2J_{HH} = 15.4 Hz, 2J_{HP} = 5 Hz, 4J_{HP} = 4.1 Hz); 3.60 (bs, 2H, Cp); 4.43 (bs, 2H, Cp); 6.71-7.63 (m, 20H, aromat. H).
13C{1H}-NMR: d = 33.8 (t, CH₃, 3J_{CP} = 11.3 Hz); 35.0 (pt, CH₂P, 1J_{CP} = 10.3 Hz, 3J_{CP} = 10.3 Hz); 38.8 (CH₂Cp); 45.2 (bs, C_{q}); 75.1, 83.4 (Cᵢₚₛₒ), 91.2 (Cp); 127.0-145.9 (m, aromat. C). 31P{1H}-NMR: d = 86.3. IR (THF): n_{CO} = 1829 cm-1. MS (FAB); *m*/*z* (%) [Frag.]: 586 (30%) [M+]; 558 (100%) [M+-CO]. C₃₅H₃₃MnOP₂ (586.532): ber. C 71.67, H 5.67, Mn 9.37, O 2.73, P 9.21; gef. C 71.82, H 6.11.

### Beispiel 21: Allgemeine Arbeitsvorschrift zur Herstellung der deprotonierten Liganden 5a-c:

Zu einer etwa 0.05 M Lösung der Liganden **5a-c** in THF wurde bei 0°C ein Äquivalent n-BuLi-Lösung in Hexan (ca. 2.3 M) zugespritzt und die entstandenen leicht gelben Lösungen mindestens eine halbe Stunde nachgerührt.

### Beispiel 22: 2,2-Bis(diphenylphosphanomethyl)-η⁵-cyclopentadienylpropyl-Eisen(II)-chlorid (14c)

In einem 100 ml Schlenkrohr wurden 0.13 g (1.02 mmol) FeCl₂ in 30 ml THF suspendiert und 15 min bei Raumtemperatur gerührt. Anschließend wurde ein Äquivalent einer Lösung von deprotoniertem **5a** über 5 min mittels einer Spritze langsam zugetropft. Die anfängliche rot-violette Farbe der Suspension ging dabei in ein tiefes Blau über.

Nach zwei Stunden Rühren bei Raumtemperatur wurde die Reaktionsmischung bis zur Trockene im Ölpumpenvakuum eingeengt. Der Rückstand wurde über 10 cm Kieselgel mit einem Lösungsmittelgemisch Et₂O/CH₂Cl₂ im Verhältnis 4:1 filtriert. Nach Entfernen des Lösungsmittels erhielt man 480 mg (81%) **14c** in Form eines blauen Pulvers. Durch Gasphasendiffusion von Et₂O in eine konzentrierte Lösung des Komplexes in CH₂Cl₂ können nach 5 Tagen tiefblaue Einkristalle erhalten werden.
1H-NMR: 1.18 (s, 2H, CH₂Cp); 1.46 (bs, 3H, CH₃); 2.25 (m, 2H, CH₂ₐP, 2J_{HH} = 6.9 Hz, 2J_{HP} = 4J_{HP} = 5 Hz), 2.54 (m, 2H, CH_{2b}P, 2J_{HH} = 6.9 Hz ,2J_{HP} = 4J_{HP} = 5 Hz); 3.92 (bs, 2H, Cp); 4.95 (t, 2H, Cp, 3J_{HP} = 1.9 Hz); 6.62-8.25 (m, 20H, aromat. H). 13C{1H}-NMR: 38.8 (t, CH₃, 3J_{CP} = 6.9 Hz); 34.5 (t, CH₂P, 1J_{CP} = 10.2 Hz); 37.1 (s, CH₂Cp); 45.4 (s, C_{q}); 59.7 (Cᵢₚₛₒ), 83.6, 89.1 (3s, Cp); 127.5-144.9 (m, aromat. C). 31P{1H}-NMR: 46.1 (bs). UV/VIS (CH₂Cl₂): 402 sh (600); 530 sh (706); 583 (848). MS (FAB); m/z (%) [Frag.]: 610 (100%) [M+]; 575 (25%) [M+-Cl]; 504 (25%) [M+-Fe-Cl]. CV (CH₃CN): E_{1/2} = -176 mV; DE = 74 mV. C₃₄H₃₃P₂FeCl (594.89): ber. C 68.63, H 5.55, P 10.43, Cl 5.97, Fe 9.42; gef. C 67.96, H 5.93.

### Beispiel 23: 2-Diphenylphosphanomethyl-2-di(m-xylyl)-phosphanomethyl-η⁵-cyclopentadienylpropyl-Eisen(II)-chlorid (14d)

Die Darstellung erfolgte analog der von **14c** aus FeCl₂: Der Ansatz, 0.09 g (0.71 mmol) Eisen(II)-chlorid suspendiert in 30 ml THF und ein Äquivalent einer Lösung von deprotoniertem **5b**, ergab nach Entfernen des Lösungsmittels das Rohprodukt von **14d**. Das Rohprodukt wurde über 6 cm Kieselgel mit einem Lösungsmittelgemisch Et₂O/CH₂Cl₂ im Verhältnis 7:1 filtriert. Nach Abziehen des Lösungsmittels im Ölpumpenvakuum erhielt man 300 mg (60%) **14d** als blaues Pulver. Durch Gasphasendiffusion von PE in eine konzentrierte und mit Et₂O überschichtete Toluollösung des Komplexes konnten nach 7 Tagen tiefblaue Einkristalle erhalten werden.
1H-NMR: 1.15 (bs, 2H, CH₂Cp); 1.47 (bs, 3H, CH₃); 1.92 (s, 6H, Xylyl-CH₃); 2.20-2.59 (2m, 4H, CH_{2a,b}PPh₂, CH_{2a,b}P(m-Xyl)₂); 2.40 (s, 6H, Xylyl-CH₃); 3.92 (bs, 2H, Cp); 4.93 (bs, 2H, Cp); 6.39-8.26 (m, 16H, aromat. H). 31P{1H}-NMR (223 K): 47.9 (bs). MS (FAB); m/z (%) [Frag.]: 650 (100%) [M+]; 615 (30%) [M+-Cl]. CV (CH₃CN): E_{1/2} = -212 mV; DE = 67 mV. C₃₈H₄₁P₂FeCl (650.997): ber. C 70.14, H 6.36, P 9.53, Cl 5.37, Fe 8.60; gef. C 70.32, H 6.85.

### Beispiel 24: 2-Diethylphosphanomethyl-2-diphenylphosphanomethyl-R⁵-cyclopentadienylpropyl-Eisen(II)-chlorid (14e)

Die Darstellung erfolgte analog der von **14c** aus FeCl₂: Der Ansatz, 0.07 g (0.58 mmol) Eisen(II)-chlorid suspendiert in 30 ml THF und ein Äquivalent einer Lösung von deprotoniertem **5c**, ergab nach Entfernen des Lösungsmittels das Rohprodukt von **14e**. Das Rohprodukt wurde über 5 cm Kieselgel mit einem Lösungsmittelgemisch Et₂O/CH₂Cl₂ im Verhältnis 3:1 filtriert. Nach Abziehen des Lösungsmittels im Ölpumpenvakuum erhielt man 176 mg (61%) **14e** als blaues Pulver. Durch Gasphasendiffusion von Et₂O in eine konzentrierte CH₂Cl₂-Lösung des Komplexes können nach 5 Tagen tiefblaue Einkristalle erhalten werden.
1H-NMR: 0.69 (bs, 2H, CH₂Cp); 1.0-1.23 (m, 6H, PCH₂CH₃); 1.31 (s, 3H, C_{q}CH₃); 1.52 (bs, 4H, PCH₂CH₃); 1.83-2.71 (m, 4H, CH_{2a,b}PPh₂, CH_{2a,b}PEt₂); 3.71 (bs, 1H, Cp); 4.57 (bs, 1H, Cp); 4.93 (bs, 2H, Cp); 7.20-8.33 (m, 10H, aromat. H). 13C-NMR (193 K): 7.0, 8.7 (2s, PCH₂CH₃); 15.3 (d, PCH₂CH₃, 1J_{CP} = 12.8 Hz); 25.2 (d, PCH₂CH₃, 1J_{CP} = 20.2 Hz); 33.4 (m, CH₂P); 33.6 (t, C_{q}CH₃, 3J_{CP} = 9.2 Hz); 36.4 (s, CH₂Cp); 44.5 (s, C_{q}); 58.0 (Cᵢₚₛₒ), 85.6, 87.3 (3bs, Cp); 128.3-144.0 (m, aromat. C). 31P{1H}-NMR (193 K): 49.4 (d, PPh₂, 2J_{PP} = 95 Hz); 56.1 (d, PEt₂, 2J_{PP} = 95 Hz). CV (CH₃CN): E_{1/2} = -292 mV; DE = 67 mV. MS (FAB): m/z (%) [Frag.]: 498 (100%) [M+]; 463 (12%) [M+-Cl]. C₂₆H₃₃P₂FeCl (498.798): ber. C 62.61, H 6.67, Cl 7.11, Fe 11.19, P 12.42; gef. C 62.02, H 6.71.

### Beispiel 25: 2,2-Bis(diphenylphosphanomethyl)-η⁵-cyclopentadienylpropyl-Eisen(III)-chlorid-hexafluorophosphat (14f)

### Methode A: Darstellung aus FeCl₃

In einem 100 ml Schlenkrohr wurde eine Lösung von 0.13 g (0.77 mmol) FeCl₃ in 10 ml THF zu einem Äquivalent einer Lösung von deprotoniertem **5a** bei Raumtemperatur während 5 min zugespritzt. Die anfängliche blaue Farbe der Reaktionslösung ging nach etwa einem Drittel der Zugabe in ein Rostrot über. Nach dreistündigem Rühren bei Raumtemperatur wurde das Lösungsmittel im Ölpumpenvakuum entfernt. Der Rückstand wurde in 10 ml eines Lösungsmittelgemischs CH₂Cl₂/THF 3:1 aufgenommen und über 5 cm Kieselgel in einer G3-Umkehrfritte filtriert. Nach Entfernen des Lösungsmittels wurde der Rückstand in 30 ml EtOH aufgenommen und zum Umsalzen mit 0.13 g (0.77 mmol) NaPF₆ versetzt. Nach einer halben Stunde Rühren wurde das Lösungsmittel erneut entfernt und der verbleibende Rückstand in 10 ml CH₂Cl₂ aufgenommen. Filtrieren über 5cm Kieselgel und Einengen des Filtrats zur Trockene ergaben 360 mg (6496) **14f** als rostroten Feststoff.
ESR: g = 2.12 (298 K); gₓ 2.21, g_{y} = 2.11, g_{z} 2.03 (100 K).
MS (FAB); m/z (%) [Frag.]: 594 (100%) [M⁺]; 559 (20%) [M⁺-Cl]; 504 (50%) [M⁺-Fe-Cl]. CV (CH₃CN): rev. Red.: E_{1/2} = -171 mV; DE = 72 mV. C₃₄H₃₃F₆P₃FeCl_{*}1.5 CH₂Cl₂ (867.245); ber. C 49.13, H 4.15, Cl 16.38, Fe 6.46, F 13.15, P 10.73; gef. C 48.99, H 4.38.

### Methode B: Darstellung durch Oxidation von 14c

Im einem 100 ml Schlenkrohr mit Septum wurden 0.24 g (0.4 mmol) **14c** in 20 ml CH₂Cl₂ gelöst und auf -70°C gekühlt. Bei dieser Temperatur wurde eine Lösung von 0.16 g (0.4 mmol) Ph₃CPF₆ in 10 ml CH₂Cl₂ über 2 min zugespritzt. Die Farbe der Lösung ging dabei von Blau über Violett in ein Rostrot über.

Das Kühlbad wurde entfernt und die Lösung noch weitere zwei Stunden gerührt. Nach Entfernen des Lösungsmittels wurde der Rückstand mit einem Lösungsmittelgemisch CH₂Cl₂ /THF 3:1 über 5 cm Kieselgel in einer G3-Umkehrfritte filtriert. Erneutes Entfernen des Lösungsmittels ergaben 0.21g (70%) **14f** als rostrotes Pulver. ESR-Spektrum und Massenspektrum stimmen mit denen aus Darstellung A) überein.

### Beispiel 26: Reduktion von 14f

### Methode A: Reduktion mit Na/Hg

In einem 100 ml Schlenkrohr wurde eine Lösung von 0.16 g (0.98 mmol) FeCl₃ in 10 ml THF zu einem Äquivalent einer Lösung von deprotoniertem **5a** bei Raumtemperatur während 5 min zugespritzt. Die Lösung wurde zwei Stunden bei dieser Temperatur gerührt. Anschließend wurde die Reaktionslösung in ein 100 ml Schlenkrohr überführt, in dem vorher 0.05 g Natrium in 2 ml Quecksilber gelöst wurden. Nach etwa 10 min nimmt die Suspension eine dunkelblaue Farbe an und man filtriert die Lösung über eine G3-Umkehrfritte. Das Lösungsmittel wurde vollständig entfernt und der Rückstand über 10 cm Kieselgel in einer G3-Umkehrfritte mit einem Lösungsmittelgemisch Et₂O/CH₂Cl₂ im Verhältnis 4/1 filtriert. Nach Abziehen des Lösungsmittels im Ölpumpenvakuum erhielt man 0.29 g (48%) eines blauen Pulvers. Das ¹H-NMR-Spektrum und Massenspektrum stimmte mit dem von **14c** überein.

### b) Reduktion mit Phenyllithium:

In einem 100 ml Schlenkrohr wurde eine Lösung von 0.1 g (0.62 mmol) FeCl₃ in 10 ml THF zu einem Äquivalent einer Lösung von deprotoniertem **5a** bei Raumtemperatur während 5 min zugespritzt. Die rostrote Lösung wurde zwei Stunden bei dieser Temperatur gerührt. Anschließend wurden zu dieser Lösung 0.33 ml (0.66 mmol) einer 2M Lösung von Phenyllithium in Cyclohexan/Et₂O während 1 min zugespritzt. Das Kühlbad wurde entfernt und die Raktionsmischung eine Stunde nachgerührt. Die Lösung färbt sich während dieser Zeit dunkelblau. Das Lösungsmittel wurde vollständig entfernt und der Rückstand über 10 cm Kieselgel in einer G3-Umkehrfritte mit einem Lösungsmittelgemisch Et₂O/CH₂Cl₂ im Verhältnis 4/1 filtriert. Nach Entfernen des Lösungsmittels erhielt man 0.31 g (52%) eines blauen Pulvers. Das ¹H-NMR-Spektrum und Massenspektrum stimmte mit dem von **14c** überein.

### Beispiel 27: 2,2-Bis(diphenylphosphanomethyl)-η⁵-cyclopentadienylpropyl-phenyl-Eisen(II) (14g)

In einem 100 ml Schlenkrohr mit Septum wurden 0.27 g (0.45 mmol) **14c** in 20 ml THF gelöst und auf -70°C gekühlt. Zu dieser blauen Lösung wurden 0.23 ml (0.46 mmol) einer 2M Lösung von Phenyllithium in Cyclohexan/Et₂O während 1 min zugespritzt. Anschließend wurde das Kühlbad entfernt und zwei Stunden nachgerüht. Die Lösung färbte sich während dieser Zeit rot-violett.

Das Lösungsmittel wurde im Ölpumpenvakuum entfernt und der Rückstand mit einem Lösungsmittelgemisch Et₂O/CH₂Cl₂ über 5 cm Kieselgur in einer G3-Umkehrfritte filtriert. Das Filtrat wurde im Ölpumpenvakuum eingeengt, wobei erneut ein farbloser Niederschlag ausfällt. Der Rückstand wurde mit Et₂O extrahiert und die erhaltene rote Lösung im Ölpumpenvakuum vollständig vom Lösungsmittel befreit. Man erhielt 0.20 g (69 %) **14g** als rotes Pulver.
1H-NMR: 1.49 (bs, 3H, CH₃); 1.98 (bs, 2H, CH₂Cp); 2.20 (m, 2H, CH₂ₐP, 2J_{HH} = 14.2 Hz, 2J_{HP} = 4.6 Hz, 4J_{HP} = 4.4 Hz); 2.40 (m, 2H, CH_{2b}P, 2J_{HH} = 13.2 Hz, 2J_{HP} = 4.6 Hz, 4J_{HP} = 4.4 Hz); 3.99 (bs, 2H, Cp); 4.25 (bs, 2H, Cp); 6.45-8.05 (m, 25H, aromat. H).
13C{1H}-NMR: 33.7 (t, CH₃); 38.6 (s, CH₂Cp); 40.0 (pt, CH₂P, 1J_{CP} = 8.5 Hz, 3J_{CP} = 7.8 Hz); 45.2 (t, C_{q}, 2J_{CP} = 2.2 Hz); 75.7 (Cᵢₚₛₒ), 80.7, 88.9 (3s, Cp); 119.8 (s, Cₚₐᵣₐ von Ph-Fe); 124.1 -145.3 (m, aromat. C); 150.1 (s, Cᵢₚₛₒ von Ph-Fe). 31P{1H}-NMR: 60.4 (s). MS (FAB); m/z (%) [Frag.]: 636 (25%) [M⁺]; 581 (100%) [M+-Fe]; 503 (55%) [M+-Fe-C₆H₅]. C₄₀H₃₈FeP₂ (636.536): ber. C 75.45, H 6.02, Fe 8.79, P 9.74; gef. C 74.32, H 6.46.

### Beispiel 28: 2,2-Bis(diphenylphosphanomethyl)-η⁵-cyclopentadienylpropyl-Eisen(II)-acetonitril-hexafluorophosphat (14h)

In einem 100 ml Schlenkrohr wurden 0.30 g (0.5 mmol) **14c** in 20 ml CH₃CN gelöst. Zu dieser dunkelblauen Lösung gab man 0.09 g (0. 53mmol) NaPF₆ und ließ 8 Stunden bei Raumtemperatur rühren. Die Farbe der Reaktionsösung änderte sich dabei von Blau nach leuchtend Rot. Das Lösungsmittel wurde im Ölpumpenvakuum entfernt, der Rückstand in 10 ml CH₂Cl₂ aufgenommen und über 5 cm Kieselgel filtriert. Das rote Filtrat wurde im Ölpumpenvakuum zur Trockene eingeengt. Man erhielt 0.28 g (75%) **14 h** als rotes Pulver.
1H-NMR: 1.52 (bs, 2H, CH₂Cp); 1.58 (t, 3H, CH₃C_{q}, 4J_{HP} = 3.1 Hz); 2.32 (m, 2H, CH₂ₐP, 2J_{HH} = 15.6 Hz, 2J_{HP} = 4.6 Hz, 4J_{HP} = 4.5 Hz); 2.54 (s, 3H, CH₃CN); 2.64 (m, 2H, CH_{2b}P, 2J_{HH} = 15.6 Hz, 2J_{HP} = 4.6 Hz, 4J_{HP} = 4.5 Hz); 4.16 (m, 2H, Cp); 5.08 (m, 2H, Cp); 6.74-7.92 (m, 20H, aromat. H). 31P{1H}-NMR: 52.5 (s, CH₂P); -144.3 (sept, PF₆). 13C{1H}-NMR: 6.7 (s, CH₃CN); 33.2-33.8 (m, CH₃, CH₂P); 36.3 (s, CH₂Cp); 46.0 (s, C_{q}); 68.8 (Cᵢₚₛₒ), 80.7, 92.3 (3s, Cp); 128.5-143.3 (m, CH₃CN, aromat. C). MS (FAB); m/z (%) [Frag.]: 600 (20%) [M+], 559 (100%) [M+-CH₃CN]; 503 (70%) [M+-Ph]. IR (CsI): 2255 cm-1 (w). CV (CH₃CN): rev. Ox.: E_{1/2} = 535 mV; DE = 70 mV. C₃₆H₃₆F₆FeNP₃ (745.446): ber. C 57.99, H 4.83, F 15.30, Fe 7.52, N 1.88, P 12.48; gef. C 58.38, H 5.14, N 1.39, P 12.26.

### Beispiel 29: Allgemeine Arbeitsvorschrift zur Herstellung von Lithiumdiarylphosphiden und Lithiumdibenzophospholid

Zu einer etwa 0.3 M Lösung des Diarylphosphans bzw. 5-H-Dibenzophosphols in THF wurde bei 0°C in etwa 5 min ein Äquivalent n-BuLi-Lösung in Hexan (ca. 2.3 M) getropft. Anschließend ließ man die Lösung noch mindestens eine halbe Stunde nachrühren. Die so hergestellten roten Lösungen wurden unmittelbar zur Synthese eingesetzt.

### Beispiel 30: 3-(Cyclopentadienylmethyl)-3-(diphenylphosphanomethyl)-oxetan (2a)

In einem 250 ml Schlenkrohr mit Septum wurden 2.6 g (10 mmol) **1a** in 50 ml THF gelöst und die Lösung auf -5°C gekühlt. Bei dieser Temperatur wurde über einen Kapillarschlauch ein Äquivalent einer Diphenylphosphidlösung während zwei Stunden zur Oxetanlösung zugetropft. Nach beendeter Zugabe wurde noch eine halbe Stunde bei Raumtemperatur nachgerührt.

Während dieser Zeit wurden in einem 250 ml Dreihalskolben mit Rückflußkühler, Septum und Inertgasanschluß 5.2 g (17 mmol) CpMgCl_{*}2THF in 50 ml THF gelöst und auf 60°C erwärmt. Wiederum wurde über einen Kapillarschlauch die Lösung des Oxetans während 45 min zugetropft. Nach beendeter Zugabe wurde die Reaktionslösung noch drei Stunden gekocht

Die Aufarbeitung erfolgt durch Zugabe von 100 ml 10%-Ammoniumchloridlösung. Nach Abtrennen der organischen Phase wurde die wäßrige Phase noch zweimal mit jeweils 30 ml Diethylether extrahiert und die vereinigten organischen Phasen anschließend mit Kochsalz-Lösung bis zur pH-Neutralität gewaschen. Nach Entfernen des Lösungsmittels wurde der erhaltene zähe, farblose Rückstand in Methylenchlorid gelöst und auf Kieselgur aufgezogen. Blitzchromatographie an einer Kieselgelsäule (25 _{*} 4 cm; Eluens: PE/THF im Verhältnis 9: 1; R_{F} = 0.38) ergeben nach Einengen der Produktfraktion im Ölpumpenvakuum 2 g (60 %) **2a** in Form eines farblosen Öls.
MS (EI); *m*/*z* (%) [Frag.]: 334 (98%) [ M⁺]; 303 (75%) [ M⁺-CH₂O-H]; 183 (100%) [ PPh₂⁺-2H]. C₂₂H₂₂OP (333.390): ber. C 79.04, H 6.89, O 4.79, P 9.28; gef. C 74.27, H 6.88.

### Beispiel 31: 2,2-Bis(diphenylphosphanomethyl)-3-cyclopentadienyl-1-propanol (3a)

In einem 250 ml Dreihalskolben mit Rückflußkühler, Septum und Inertgasanschluß wurden 2 g (6 mmol) **2a** in 50 ml THF gelöst und bei 0°C durch Zuspritzen von 2.5 ml (6 mmol) n-BuLi-Lösung deprotoniert. Es wurde eine halbe Stunde nachgerührt. Über einen Kapillarschlauch wurden 1.2 Äquivalente einer Diphenylphosphidlösung bei Raumtemperatur innerhalb einer halben Stunde zugetropft. Die Reaktionslösung wurde anschließend drei Stunden unter Rückfluß erhitzt, wobei die rote Färbung langsam einer orangenen Färbung wich.

Die Reaktionsmischung wurde im Ölpumpenvakuum eingeengt und der Rückstand mit 50 ml Diethylether aufgenommen. Zur Hydrolyse gab man 20 ml Wasser zu und rührte 10 min. Die organische Phase wurde abgetrennt und die wäßrige Phase zweimal mit 30 ml Diethylether extrahiert. Die vereinigten Etherphasen wurden mit Kochsalzlösung zur pH-Neutralität gewaschen und über Natriumsulfat getrocknet. Das verbleibende farblose Öl wurde in Methylenchlorid gelöst und auf Kieselgur aufgezogen. Es folgte Blitzchromatographie an Kieselgel (25 _{*} 4 cm; Eluens: PE/Diethylether im Verhältnis 8.5: 1.5; R_{F} = 0.26). Man erhielt 1.3 g (42%) **3a** als farbloses, zähes Öl.
MS (EI); *m*/*z* (%) [Frag.]: 520 (60%) [M⁺]; 443 (100%) [M⁺-C₆H₅]; 335 (75%) [M⁺-PPh₂]; 185 (30%) [PPh₂^{**+**}]; 183 (65%) [PPh₂⁺-2H]. C₃₄H₃₄OP₂ (520.591): ber. C 78.46, H 6.54, O 3.08, P 11.92; gef. C 76.17, H 6.73.

### Beispiel 32: 3-Cyclopentadienyl-2-(di-m-xylylphosphanomethyl)-2-(diphenylphosphanomethyl)-1-propanol (3b)

Die Darstellung erfolgte analog der von **3a**. Der Ansatz, 1.4 g (4.2 mmol) **2**, deprotoniert mit 1.8 ml (4.2 mmol) n-BuLi-Lösung und 1.2 Äquivalenten Lithiumdi-m-xylylphosphid-Lösung lieferte das Rohprodukt von **3b**. Das verbleibende farblose Öl wurde in Methylenchlorid gelöst und auf Kieselgur aufgezogen. Es folgte Blitzchromatographie an Kieselgel (20 * 4 cm; Eluens: PE/Diethylether im Verhältnis 8.75: 1.25; R_{F} = 0.31). Man erhielt 1.15 g (47%) **3b** als ein farbloses, zähes Öl.
MS (EI); *m*/*z* (%) [Frag.]: 576 (83%) [M⁺]; 499 (100%) [M⁺-C₆H₅]; 471 (75%) [M⁺-C₆H₅-CH₂O]; 391 (90%) [M⁺-PPh₂]; 241 (65%) [P(m-Xyl)₂⁺]; 185 (20%) [PPh₂⁺]. C₃₈H₄₂OP₂ (576.689): ber. C 79.17, H 7.29, O 2.78, P 10.76; gef. C 78.34, H 7.55.

### Beispiel 33: 3-Cyclopentadienyl-2-(5-dibenzophospholylmethyl)-2-(diphenylphosphanomethyl)-1-propanol (3c)

Die Darstellung erfolgte analog der von **3a**. Der Ansatz, 1.6 g (4.8 mmol) **2a**, deprotoniert mit 2 ml (4.8 mmol) n-BuLi-Lösung und 1.2 Äquivalenten Lithiumdibenzophospholid-Lösung lieferte das Rohprodukt von **3c**. Das verbleibende farblose Öl wurde in Methylenchlorid gelöst und auf Kieselgur aufgezogen. Es folgte Blitzchromatographie an Kieselgel (23 _{*} 4 cm; Eluens: PE/Diethylether im Verhältnis 8.5: 1.5, RF = 0.27). Man erhielt 1.36 g (55%) **3c** in Form eines farblosen, zähen Öls.
MS (EI); *m*/*z* (%) [Frag.]: 518 (40%) [M⁺];439 (20%) [M⁺-C₆H₅]; 335 (60%) [M⁺-DBP]; 183 (100%) [DBP⁺]. C₃₄H₃₂OP₂ (518.575): ber. C 78.75, H 6.22, O 3.09, P 11.95; gef. C 77.08, H 6.56.

### Beispiel 34: Essigsäure-2,2-bis(diphenylphosphanomethyl)-3-cyclopentadienyl-1-propylester (4a)

In einem 250 ml Dreihalskolben mit Rückflußkühler, Septum und Inertgasanschluß wurden 4.26 g (12.5 mmol) **2a**, in 50 ml THF gelöst und bei 0°C durch Zuspritzen von 5.2 ml (12.5 mmol) n-BuLi-Lösung deprotoniert. Es wurde eine halbe Stunde nachgerührt. Über einen Kapillarschlauch wurden 1.2 Äquivalente einer Diphenylphosphidlösung bei Raumtemperatur innerhalb einer halben Stunde zugetropft. Nach Entfernen des Lösungsmittels wurde das Rohprodukt in 50 ml Toluol aufgenommen und zu dieser orange-roten Lösung bei Raumtemperatur 1.6 ml (22.8 mmol) Acetylchlorid langsam zugespritzt, wobei ein farbloser Niederschlag die Esterbildung anzeigte. Die Lösung erwärmte sich dabei und verfärbte sich rot.

Hydrolyse erfolgte durch Zugabe von 100 ml entgastem Wasser mit 1ml Pyridin. Es wurde 10 min nachgerührt und die organische Phase nach Phasentrennung abgetrennt. Nach zwemaligem Extrahieren der wäßrigen Phase mit 50 ml Toluol wurden die vereinigten Toluolphasen mit Kochsalzlösung zur pH-Neutralität gewaschen und über Natriumsulfat getrocknet. Die Lösung wurde weitgehend vom Lösungsmittel befreit und das erhaltene zähe Öl in Methylenchlorid aufgenommen. Aufziehen auf Kieselgur und Blitzchromatographie an Kieselgel (25 _{*} 4 cm; Eluens: PE/Diethylether im Verhältnis 8.5: 1.5; R_{F} = 0.26) ergeben 2.4 g (34 %) **4a** in Form eines farblosen Öls.
MS (EI); *m*/*z* (%) [Frag.]: 562 (63%) [M⁺]; 485 (100%) [M⁺-C₆H₅]; 377 (95%) [M⁺-PPh₂]; 183 (65%) [PPh₂⁺-2H]. C₃₆H₃₆O₂P₂ (562.628): ber. C 76.87, H 6.41, O 5.69, P 11.03; gef. C 75.93, H 6.56.

### Beispiel 35: 3-Benzylthiomethyl-3-cyclopentadienyl-oxetan (1b)

In einem ausgeheizten 250 ml Schlenkrohr mit Septum wurde zunächst die Benzylthiolatlösung bereitet. Dazu wurden 2.00 g (16.1 mmol) Benzylthiol in 50 ml THF gelöst und bei 0°C durch Zuspritzen von 6.7 ml (16.1 mmol) n-BuLi-Lösung deprotoniert. Die Lösung wurde nun mindestens eine halbe Stunde bei Raumtemperatur gerührt. Unter dessen wurden in einem weiteren 250 ml-Schlenkrohr mit Septum 4.37 g (16.89 mmol) **1a** in 50 ml THF gelöst und die Lösung auf -15°C gekühlt. Die Benzylthiolatlösung wurde während zwei Stunden langsam zur Oxetanlösung zugetropft. Nach beendeter Zugabe wurde noch eine halbe Stunde bei Raumtemperatur nachgerührt. Während dieser Zeit wurden in einem 250 ml Dreihalskolben mit Rückflußkühler, Septum und Inertgasanschluß 5.9 g (22 mmol) CpMgCl_{*}2THF in 50 ml THF gelöst und auf 60°C erwärmt. Die Lösung des Oxetans wurde über 45 min zur Grignard-Lösung zugetropft. Nach beendeter Zugabe wurde die Reaktionsmischung noch drei Stunden gekocht. Die Aufarbeitung erfolgt durch Zugabe von 100 ml 10%-Ammoniumchloridlösung. Nach Abtrennen der organischen Phase wurde die wäßrige Phase noch dreimal mit jeweils 30 ml Diethylether extrahiert und die vereinigten organischen Phasen anschließend mit Kochsalz-Lösung bis zur pH-Neutralität gewaschen. Im Ölpumpenvakuum wurde das Lösungsmittel abgezogen, der erhaltene zähe, farblose Rückstand in Methylenchlorid gelöst und auf Kieselgur aufgezogen. Blitzchromatographie an einer Kieselgelsäule (25 _{*} 4 cm; Eluens: PE/THF im Verhältnis 8.7: 1.3; R_{F} = 0.39) ergeben nach Einengen der Produktfraktion im Ölpumpenvakuum 3.08 g (67%) **1b** als farbloses Öl.
MS (EI); m/z (%) [Frag.]: 272 (25%) [M⁺], 181 (100%) [M⁺-CH₂Ph], 91 (50%) [CH₂Ph⁺]. C₁₇H₂₀OS (272.413).

### Beispiel 36: 2-Benzylthiomethyl-3-cyclopentadienyl-2-diphenylphosphanomethyl-1-propanol (3d)

In einem 250 ml Dreihalskolben mit Rückflußkühler, Septum und Inertgasanschluß wurden 2.77 g (10.2 mmol) **1b** in 50 ml THF gelöst und bei 0°C durch Zuspritzen von 4.4 ml (10.2 mmol) n-BuLi-Lösung deprotoniert. Es wurde eine halbe Stunde nachgerührt. Über einen Kapillarschlauch wurden 1.2 Äquivalente einer Diphenylphosphidlösung bei Raumtemperatur innerhalb einer halben Stunde zugetropft. Die Reaktionslösung wurde 16 Stunden bei dieser Temperatur gerührt und anschließend das Lösungsmittel weitgehend entfernt. Der Rückstand wurde in 50 ml Diethylether aufgenommen und mit 20 ml Wasser hydrolysiert. Die organische Phase wurde abgetrennt und die wäßrige Phase zweimal mit 30 ml Diethylether extrahiert. Die vereinigten Etherphasen wurden mit Kochsalzlösung zur pH-Neutralität gewaschen und über Natriumsulfat getrocknet. Das verbleibende farblose Öl wurde in Methylenchlorid gelöst und auf Kieselgur aufgezogen. Es folgte Blitzchromatographie an Kieselgel (25 _{*} 4 cm; Eluens: PE/Diethylether im Verhältnis 8.75: 1.25; R_{F} = 0.29). Man erhält 1.9 g (40 %) **3d** in Form eines farblosen, zähen Öls.
MS (EI);m/z (%) [Frag.]: 458 (3%) [M⁺]; 367 (100%) [M⁺-CH₂PPh₂]; 217 (25%) [SPPh₂⁺]; 183 (30%) [PPh₂⁺-2H]. C₂₉H₃₁OPS (457.598): ber. C 75.98, H 6.77, O 3.49, S 6.99, P 6.77; gef. C 74.38, H 7.10.

### Beispiel 37: Arbeitsvorschrift zur Herstellung der deprotonierten Liganden 3a und 4a:

Zu einer etwa 0.05 M Lösung der Liganden **3a** und **4a** in THF wurde bei 0°C ein Äquivalent n-BuLi-Lösung in Hexan (ca. 2.3 M) zugespritzt und die entstandenen leicht gelben Lösungen mindestens eine halbe Stunde nachgerührt.

### Beispiel 38: 2,2-Bis(diphenylphosphanomethyl)-3-η⁵-cyclopentadienyl-1-propanol-Eisen(II)-chlorid (14a)

In einem 100 ml Schlenkrohr wurden 0.32 g (0.49 mmol) Bis-triphenylphosphan-Eisen(II)-chlorid in 30 ml THF gelöst und auf -70°C gekühlt. Dazu gab man innerhalb von 2 min ein Äquivalent einer Lösung von deprotoniertem **3a**. Nach anfänglicher Rotfärbung nahm die Lösung eine schmutzig-blaue Farbe an. Man ließ 15 min bei dieser Temperatur rühren und entfernte dann das Kühlbad. Nun wurde noch weitere zwei Stunden nachgerührt. Es bildete sich während dieser Zeit ein bräunlicher Niederschlag. Das Lösungsmittel wurde entfernt und der Rückstand in eine G3-Umkehrfritte auf 5 cm Kieselgel überführt. Durch Waschen mit 30 ml PE/Diethylether 2/1 wurde eine leicht grünliche Fraktion abgetrennt. Das Produkt wurde als dunkelblaue Fraktion mit Diethylether/Methylenchlorid 4/1 eluiert. Nach Entfernen des Lösungsmittels im Ölpumpenvakuum erhielt man 0.19 g (64%) **14a** in Form eines dunkelblauen Pulvers.
1H-NMR (CH₂Cl₂): 1.19 (2H, CH₂Cp); 2.26 (m, 2H, CH₂ₐP, 2J_{HH} = 16.1 Hz); 2.77 (m, 2H, CH_{2b}P); 3.73 (bs, 2H, CH₂OH); 3.96 (bs, 2H, Cp); 4.95 (bs, 2H, Cp); 6.62-8.26 (m, 20H, aromat. H).
31P{1H}-NMR (CH₂Cl₂, 223K): 44.6 (bs). MS (FAB); m/z (%) [Frag.]: 610 (100%) [M+]; 575 (25%) [M+-Cl]. C₃₄H₃₃P₂FeCl (610.890): ber. C 66.87, H 5.45, P 10.15, O 2.62, Cl 5.73, Fe 9.18; C 65.33, H 5.56, P 9.44.

### Beispiel 39: Essigsäure-2,2-bis(diphenylphosphanomethyl)-3-η⁵-cyclopentadienyl-1-propylester-Eisen(II)-chlorid (14b)

In einem 100 ml Schlenkrohr wurden 0.4 g (0.62 mmol) Bis-triphenylphosphan-Eisen(II)-chlorid in 30 ml THF gelöst und auf -70°C gekühlt. Dazu gab man innerhalb von 2 min ein Äquivalent einer Lösung von deprotoniertem **4a**. Nach anfänglicher Rotfärbung nahm die Lösung eine blau-grüne Farbe an. Man ließ 15 min bei dieser Temperatur rühren und entfernte dann das Kühlbad. Nun wurde noch weitere zwei Stunden nachgerührt. Es bildete sich während dieser Zeit ein bräunlicher Niederschlag. Das Lösungsmittel wurde im Ölpumpenvakuum abgezogen und der Rückstand in eine G3-Umkehrfritte auf 5 cm Kieselgel überführt. Durch Waschen mit 30 ml PE/Diethylether 2/1 wurde eine leicht grünliche Fraktion abgetrennt. Das Produkt wurde als tief-blaue Fraktion mit Diethylether/Methylenchlorid 4/1 eluiert. Nach Entfernen des Lösungsmittels im Ölpumpenvakuum erhielt man 0.18 g (44%) **14b** in Form eines dunkelblauen Pulvers.
1H-NMR (CH₂Cl₂): 1.31 (2H, CH₂Cp); 2.21 (3H, CH₃CO₂); 2.26-2.34 (m, 2H, CH₂ₐP); 2.63-2.73 (m, 2H, CH_{2b}P); 3.76 (bs, 2H, CH₂O); 3.97 (bs, 2H, Cp); 4.98 (bs, 2H, Cp); 6.62-8.27 (m, 20H, aromat. H). 31P{1H}-NMR (CH₂Cl₂, 295K): 44.6 (bs). MS (FAB); m/z (%) [Frag.]: 652 (100%) [M+]; 617 (70%) [M+-Cl]. C₃₆H₃₅ClFeO₂P₂ (652.927): ber. C 66.16, H 5.38, Cl 5.48, Fe 8.58, O 4.90, P 9.50; gef. C 66.20, H 5.80, P 9.36.

### Beispiel 40: η²-2,2-Bis(diphenylphosphanomethyl)-η⁵-cyclopentadienylpropyl-dicarbonyl-Molybdän(II)-iodid (15a)

In einem 100 ml Schlenkrohr mit Septum wurden 0.30 g (0.59 mmol) **5a** in 20 ml THF gelöst und mit 0.26 ml (0.59 mmol) 2.3 M n-BuLi-Lösung bei 0°C deprotoniert. Nach Entfernen der Kühlung wurde noch 30 min nachgerührt. Anschließend wurden 1.3 ml (1.3 mmol) Boran-THF-Komplex bei Raumtemperatur zugespritzt. Die Lösung ließ man 15 min nachrühren und engte im Ölpumpenvakuum bis zu Trockene ein.

Der Rückstand wurde erneut in 30 ml THF aufgenommen und bei Raumtemperatur wurden 0.18 g (0.59 mmol) Mo(CH₃CN)₃(CO)₃ zugeben. Die anfängliche Suspension ging innerhalb von 5 Minuten in eine braune Lösung über. Diese ließ man 2 h bei Raumtemperatur rühren und tropfte unter Eiskühlung über eine Spritze eine Lösung von 0.15 g (1.3 mmol) Iod in 10 ml THF zu. Die rotbraune Reaktionsmischung ließ man 3 h rühren und entfernte im Ölpumpenvakuum das Lösungsmittel. Der verbleibende Rückstand wurde in 5 ml Toluol aufgenommen und über 5 cm Kieselgel in einer G3-Umkehrfritte filtriert. Das Produkt lief dabei als scharfe, rote Bande über das Kieselgel. Im IR-Spektrum zeigte das Produkt zwei Carbonylbanden bei n = 2038 und 1962 cm-¹.

Das Volumen der erhaltenen roten Toluollösung wurde auf die Hälfte im Ölpumpenvakuum eingeengt und 0.15 g (1.3 mmol) DABCO zugegeben. Die Lösung wurde auf 70°C im Wasserbad erhitzt und 2 h bei dieser Temperatur gerührt. Dabei bildete sich ein gelber Niederschlag. Die Reaktionsmischung wurde bis auf 5 ml eingeengt und an Kieselgel (10*2 cm) mit Toluol als Laufmittel chromatographiert. Das Produkt lief als kräftig rote Bande. Das Lösungsmittel wurde im Ölpumpenvakuum vollständig abgezogen. Man erhielt 0.38 g (76%) **15a** in Form eines roten Pulvers.
1H-NMR (CDCl₃): 0.81 (bs, 3H, CH₃); 2.10 (bs, 2H, CH₂Cp); 2.30-2.57 (m, 4H, CH₂P, CH₂PMo); 4.93, 4.99, 5.67, 5.68 (4s, 4H, Cp); 7.27-7.59 (m, 20H, aromat. H). 13C{1H}-NMR (CDCl₃): 29.7 (m, CH₃); 36.8-38.5 (m, CH₂P, CH₂PMo, C_{q}); 46.0, 46.3 (2d, CH₂Cp, 3J_{CP} = 14 Hz); 76.5 (Cᵢₚₛₒ), 77.0 (Cᵢₚₛₒ), 87.7, 88.2, 95.4, 96.0, 100.1, 100.2 (8s, Cpₐ, Cp_{b}); 128.13-140.9 (m, aromat. C). 31P{1H}-NMR (CDCl₃): 47.6 (s, MoPPh₂); -26.3 (s, PPh₂). IR (Toluol): 1964 (s); 1959 (sh); 1887 (vs); 1859 (sh) cm-1. MS (FAB); m/z (%) [Frag.]: 783 (8%) [M+]; 728 (100%) [M+-2CO]; 651 (30%) [M+-I]. C₃₆H₃₃IMoO₂P₂ (782.442): ber. C 55.24, H 4.22, I 16.24, Mo 12.28, O 4.09, P 7.93; gef. C 54.47, H 4.95, P 7.65.

### Beispiel 41: 2,2-Bis(diphenylphosphanomethyl)-η⁵-cyclopentadienylpropyl-Molybdän(II)-dicarbonyl-iodid (15b)

In einem 100 ml Cariusrohr mit Teflonverschluß wurden 0.7 g (0.9 mmol) **15a** eingewogen und mit 40 ml Toluol aufgenommen. Das Cariusrohr wurde verschlossen und mit einer externen, kühlbaren Strahlungsquelle (Quecksilberhochdrucklampe) über 48 h bestrahlt. Während dieser Zeit fiel ein ockerfarbener Niederschlag aus. Die Lösung war am Ende der Bestrahlung leicht gelblich gefärbt. Der Niederschlag wurde in einer G3-Umkehrfritte abgetrennt, dreimal mit 10 ml THF und einmal mit 10 ml Diethylther gewaschen und im Ölpumpenvakuum getrocknet. Man erhielt 0.51 g (72%) **15b** in Form eines gelb-ockerfarbenen Pulvers.

Gelbe, für die Röntgenstrukturanalyse geeignete Einkristalle wurden durch Gasphasendiffusion von Diethylether in eine Lösung von **15b** in Methylenchlorid während 7 Tagen erhalten.
1H-NMR (CD₂Cl₂): 1.82 (bs, 3H, CH₃); 2.43 (d, 2H, CH₂ₐP, 2J_{HH} = 15.1 Hz); 2.67-2.73 (m, 4H, CH_{2b}P, CH₂Cp); 4.95, 5.60 (2bs, 2H, Cp); 6.95-7.42 (m, 20H, aromat. H). 13C{1H}-NMR (CD₂Cl₂): 34.5-35.0 (m, CH₃, CH₂P); 36.1 (s, CH₂Cp); 44.1 (t, C_{q}, 2J_{CP} = 3.7 Hz); 91.3, 98.2 (2s, Cp); 102.5 (1s, Cp, Cᵢₚₛₒ); 129.0-141.6 (m, aromat. C); 238.5 (m, CO). 31P{1H}-NMR (CD₂Cl₂): 45.2 (s).
IR (THF): 1969 (vs), 1901 (s) cm-1. MS-Kation (FAB); m/z (%) [Frag.]: 657 (100%) [M+]; 629 (15%) [M+-CO]; 601 (20%) [M+-2CO]. MS-Anion (FAB): 127 [I-]. C₃₆H₃₃IMoO₂P₂ (782.442): ber. C 55.24, H 4.22, I 16.24, Mo 12.28, O 4.09, P 7.93; gef. C 54.18, H 4.56.

### Beispiel 42: 2,2-Bis(diphenylphosphanomethyl)-η⁵-cyclopentadienylpropyl-oxo-Molybdän(IV)-iodid, (15c)

In einer Bestrahlungsapparatur wurden 0.78 g (1mmol) **15a** in 100 ml Toluol gelöst. Der verbleibende Gasraum der Apparatur wurde mit Luft vermischt und die rote Lösung unter starkem Rühren 48 h bei 20°C bestrahlt. Während dieser Zeit fiel ein rot-violetter Niederschlag aus. Der Niederschlag wurde in einer G3-Umkehrfritte abgetrennt und mit THF solange gewaschen, bis das Filtrat farblos blieb. Anschließend wurde der verbleibende Niederschlag mit einer Mischung von Methylenchlorid/THF 4/1 eluiert und das leuchtend rote Eluat im Ölpumpenvakuum vom Lösungsmittel befreit. Man erhielt 0.48 g (65%) **15c** als rot-violettes Pulver. Rot-violette Einkristalle konnten durch Gasphasendiffusion von Diethylether in eine Ethanol/Methylenchlorid-Lösung (4:1) des Komplexes bei 3°C nach 7 Tagen erhalten wurden.
1H-NMR (CD₂Cl₂): 1.75 (t, 3H, CH₃, 3J_{HP} = 3.2 Hz); 2.01 (bs, 2H, CH₂Cp); 2.50, 2.79 (2ddd, 2H, CH_{2a,b}P, 2J_{HH} = 16.3 Hz, 2J_{HP} = 4.5 Hz, 4J_{HP} = 4 Hz); 5.25 (sh, 2H, Cp); 5.98 (bs, 2H, Cp); 7.00-7.76 (m, 20H, aromat. H). 13C{1H}-NMR (CD₂Cl₂): 31.9 (t, CH₂P, 1J_{CP} = 15.2 Hz); 34.6 (bs, CH₃); 36.1 (s, CH₂Cp); 44.1 (s, C_{q}); 95.2, 107.8 (2s, Cp), 110.5 (s, Cp, Cᵢₚₛₒ); 128.8-138.5 (m, aromat. C). 31P{1H}-NMR (CD₂Cl₂): 51.6 (s). IR (CsI): nₒ= 919 (s). MS (FAB); m/z (%) [Frag.]: 617 (100%) [M+-I]; 503 (25%) [M+-I-Mo-O]. C₃₄H₃₃IMoOP₂ * CH₂Cl₂ (827.367): ber. C 50.81, H 4.26, Cl 8.57, I 15.34, Mo 11.60, O 1.93, P 7.49; gef. C 50.36, H 4.39.

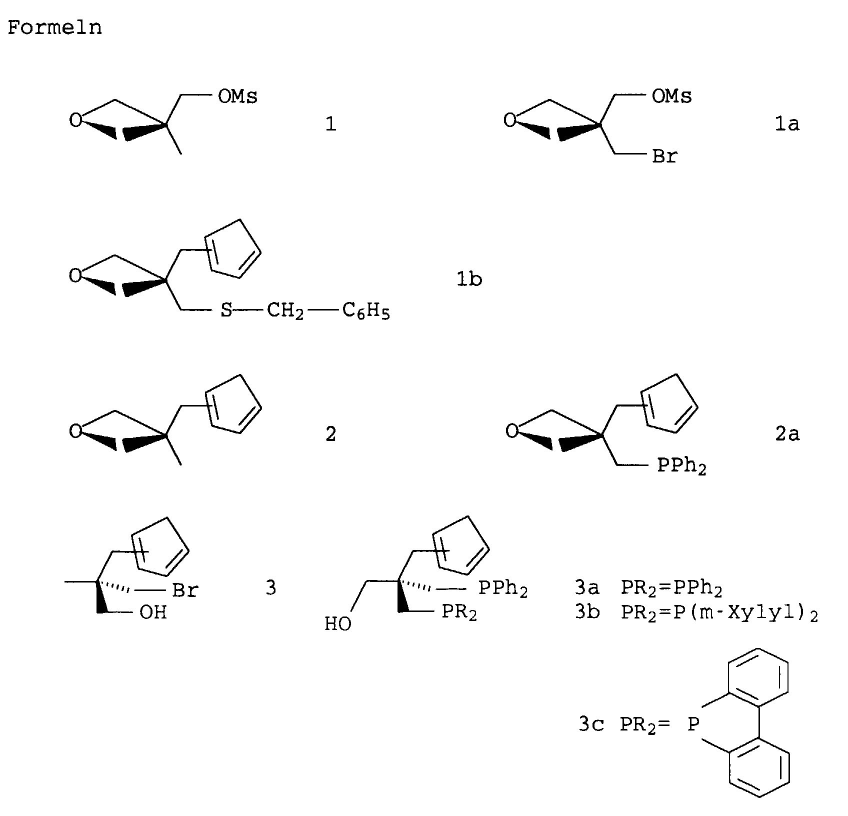

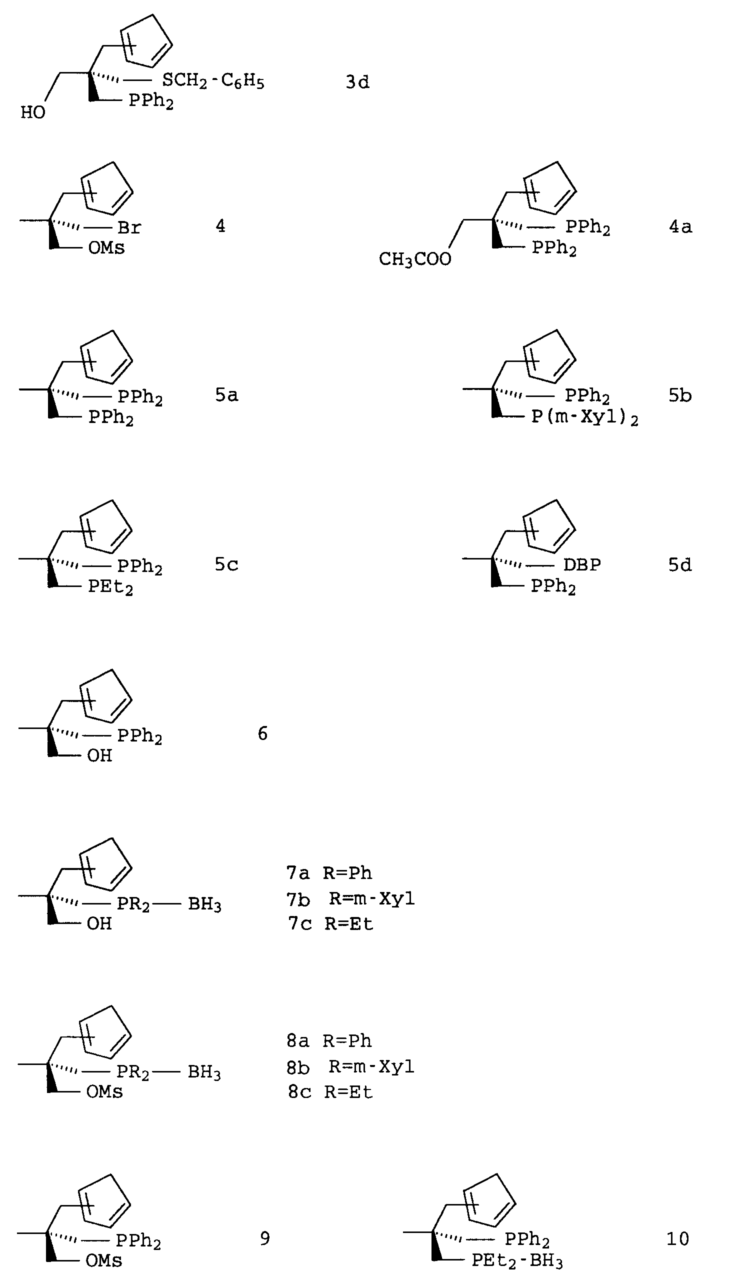

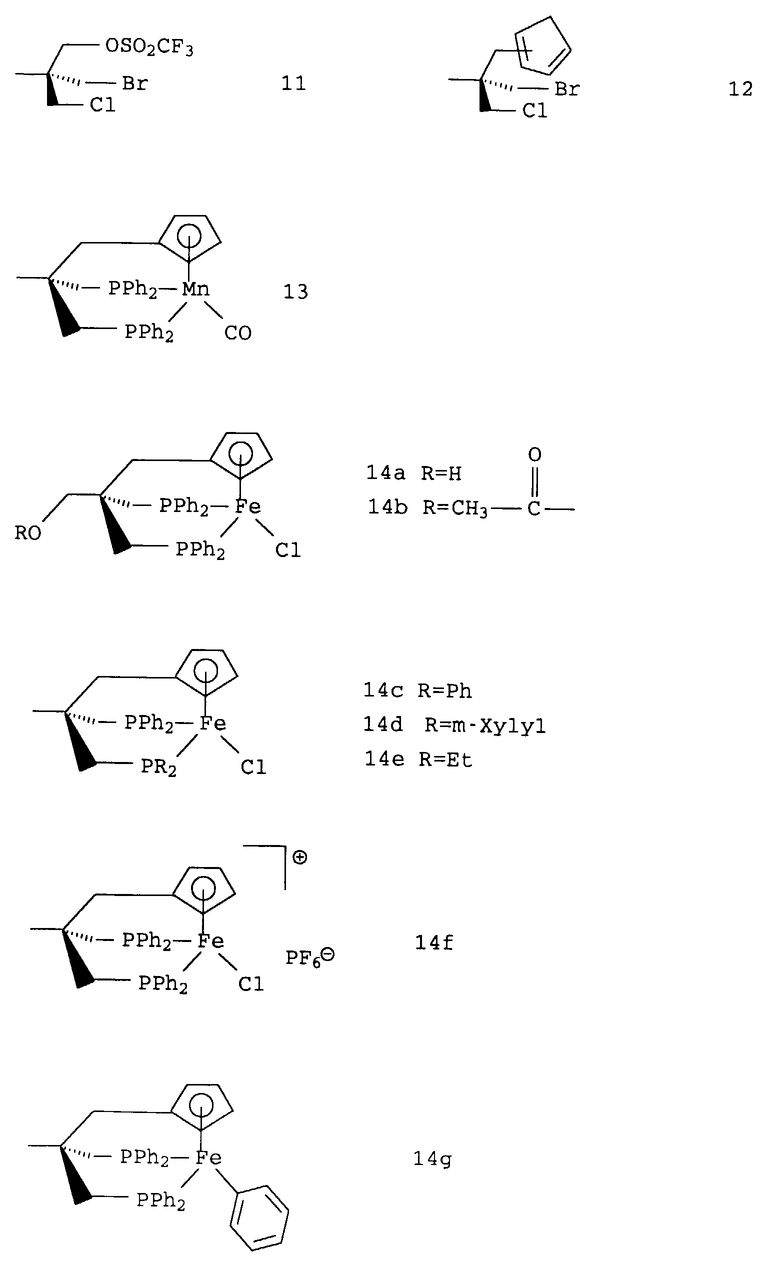

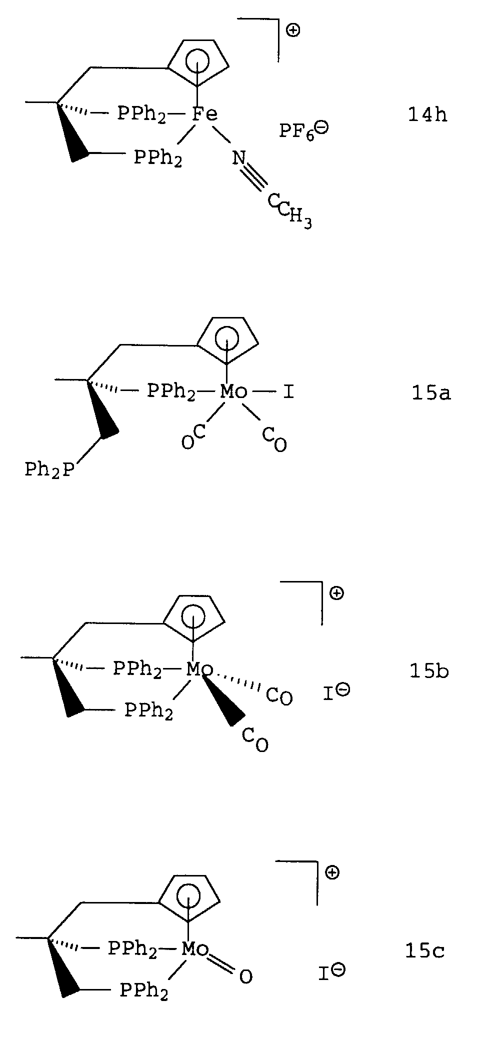

## Patentansprüche

1. Tripodale Cyclopentadienderivate der allgemeinen Formel (I) 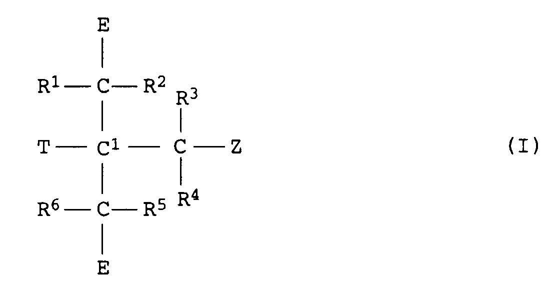 worin
E gleich oder verschieden ist und für -N(R) (R), -P(R) (R), -As(R) (R), -Sb(R) (R), -OR, -SR, -SeR, -TeR, wobei R gleich oder verschieden ist und Wasserstoff- einen C₁- bis C₂₀-kohlenstofforganischen oder C₁- bis C₃₀-siliciumorganischen Rest bedeutet, oder E für eine Abgangsgruppe X steht und
R¹, R², R³, R⁴, R⁵, R⁶ gleich oder verschieden ist und Wasserstoff einen C₁- bis C_{2O}- kohlenstofforganischen oder C₁-bis C_{3O}-siliciumorganischen Rest bedeutet,
Z für den Cyclopentadienylrest oder eine substituierte Cyclopentadienylstruktureinheit steht und
T Wasserstoff einen C₁- bis C₂₀-kohlenstofforganischen oder C₁- bis C₃₀-siliciumorganischen Rest bedeutet oder für eine Gruppe E-Y- steht, worin E für -N(R) (R), -P(R) (R), -As(R) (R), -Sb (R) (R), -OR, -SR, -SeR, -TeR oder eine Abgangsgruppe X steht, wobei R gleich oder verschieden ist und Wasserstoff einen C₁- bis C_{2O}-kohlenstofforganischen oder C₁- bis C_{3O}- siliciumorganischen Rest bedeutet und worin Y eine C₁- bis C_{2O}-organische Gruppe ist, die E mit C¹ verbindet.

2. Tripodale Cyclopentadienderivate nach Anspruch 1, worin E gleich oder verschieden ist und -P(R) (R) bedeutet.

3. Tripodale Cyclopentadienderivate nach den Ansprüchen 1 oder 2, worin Z den Cyclopentadienylrest oder einen, mit einer bis zu vier C₁- bis C₁₀-Alkylgruppen, substituierten Cyclopentadienylrest bedeutet.

4. Tripodale Cyclopentadienderivate nach den Ansprüchen 1 bis 3, worin T ein C₁- bis C₁₀-Alkylrest bedeutet und E-Y- für -CH₂-OR steht.

5. Tripodale Cyclopentadienderivate nach den Ansprüchen 1 bis 4, worin T für -CH₂-OR steht und wobei R Wasserstoff oder C₁- bis C₁₀-kohlenstofforganischer Rest bedeutet.

6. Verfahren zur Herstellung von tripodalen Cyclopentadienderivaten der allgemeinen Formel (I) 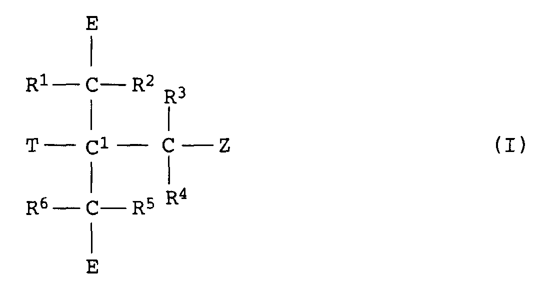 durch Umsetzung eines Oxetanderivats der allgemeinen Formel (II) 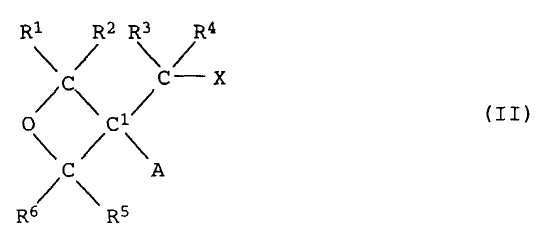 worin
R¹,R²,R³,R⁴,R⁵,R⁶ gleich oder verschieden ist und Wasserstoff, einen C₁- bis C_{2O}- kohlenstofforganischen oder C₁- bis C₃₀-siliciumorganischen Rest bedeutet,
A Wasserstoff, einen C₁- bis C₂₀-kohlenstofforganischen oder C₁- bis C_{3O}-siliciumorganischen Rest bedeutet
X eine Abgangsgruppe ist,
mit
(a) einem Cyclopentadienidanionenäquivalent oder einem substituierten Cyclopentadienidanionenäquivalent unter Substitution von X zum cyclopentadienylsubstituierten Oxetanderivat,
(b) Ringöffnung des cyclopentadienylsubstituierten Oxetanderivats mit einer Säure H-X,
(c) Umwandlung der gebildeten -OH-Funktion in eine Abgangsgruppe X und
(d) Substitution der Abgangsgruppen X durch E.

7. Verfahren zur Herstellung von tripodalen Cyclopentadienderivaten der allgemeinen Formel (I) 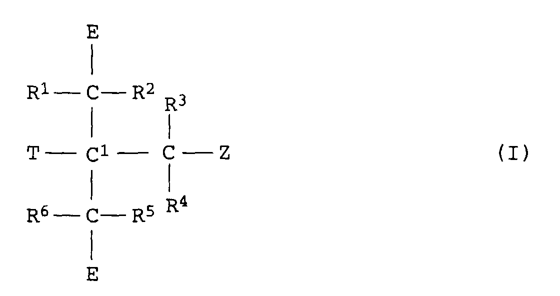 durch Umsetzung eines Oxetanderivats der allgemeinen Formel (II) 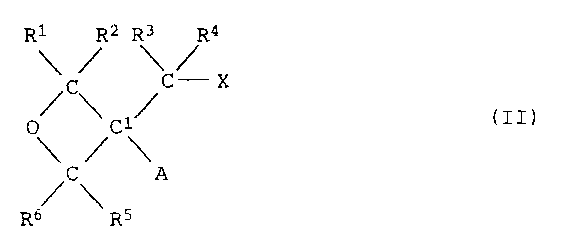 worin
R¹,R²,R³,R⁴,R⁵,R⁶ gleich oder verschieden ist und Wasserstoff, einen C₁- bis C_{2O}-kohlenstofforganischen oder C₁- bis C_{3O}-siliciumorganischen Rest bedeutet,
A Wasserstoff, einen C₁- bis C_{2O}-kohlenstofforganischen oder C₁- bis C_{3O}-siliciumorganischen Rest bedeutet und
X eine Abgangsgruppe ist,
mit
(a) einem Cyclopentadienidanionenäquivalent oder einem substituierten Cyclopentadienidanionenäquivalent unter Substitution von X zum cyclopentadienylsubstituierten Oxetanderivat,
(b) nucleophile Ringöffnung des cyclopentadienylsubstituierten Oxetanderivats mit MetEₙ, worin Met ein Element der ersten, zweiten oder dritten Hauptgruppe des Periodensystems der Elemente ist, n die maximale formale Wertigkeit von Met in der Verbindung MetEₙ bedeutet und E die für Formel (I) definierte Bedeutung hat, wobei die Abgangsgruppe X ausgeschlossen ist,
(c) Umwandlung der gebildeten OH-Funktion in eine Abgangsgruppe X,
(d) Substitution der Abgangsgruppe X durch E.

8. Verfahren zur Herstellung chiraler, tripodaler Cyclopentadienderivate nach Anspruch 7 dadurch gekennzeichnet, daß man bei (b) und (d) unterschiedliche Substituenten E einführt.

9. Verfahren zur Herstellung funktionalisierter, tripodaler Cyclopentadienderivate der allgemeinen Formel (III) 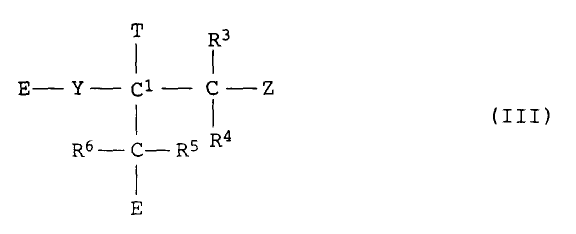 worin
E gleich oder verschieden ist und für -N(R) (R), -P(R) (R), -As(R) (R), -Sb (R) (R), -OR, -SR, -SeR, -TeR, wobei R gleich oder verschieden ist und Wasserstoff- einen C₁- bis C_{2O}-kohlenstofforganischen oder C₁- bis C_{3O}-siliciumorganischen Rest bedeutet, oder E für eine Abgangsgruppe X steht und
Y eine C₁- bis C_{2O}-organische Gruppe ist, die E mit C¹ verbindet,
R³, R⁴, R⁵, R⁶ gleich oder verschieden sind und Wasserstoffeinen C₁- bis C_{2O}-kohlenstofforganischen oder C₁- bis C_{3O}-silciumorganischen Rest bedeutet,
Z für den Cyclopentadienylrest oder eine substituierte Cyclopentadienylstruktureinheit steht und
T für RO-C(R¹) (R²)- steht, worin R, R¹ und R² gleich oder verschieden ist und Wasserstoff oder C₁- bis C₁₀-kohlenstofforganischer Rest bedeuten,
durch Umsetzung eines Oxetanderivats der Formel (II) 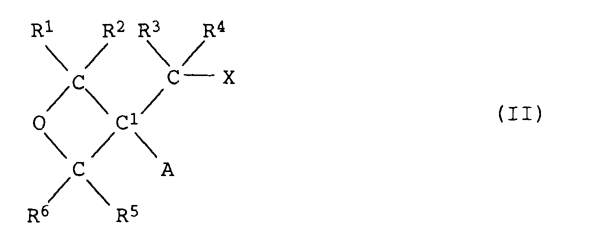 worin
R¹, R², R³, R⁴, R⁵, R6 gleich oder verschieden ist und Wasserstoff einen C₁- bis C_{2O}- kohlenstofforganischen oder C₁- bis C₃₀-siliciumorganischen Rest bedeutet,
A eine Struktureinheit -Y-X bedeutet, worin X eine Abgangsgruppe und Y eine C₁- bis C_{2O}-organische Gruppe ist, die X mit C¹ verbindet,
X eine Abgangsgruppe ist
mit
(a) MetEₙ
worin Met ein Element der ersten, zweiten oder dritten Hauptgruppe des Periodensystems der Elemente ist, n die maximale formale Wertigkeit von Met in der Verbindung MetEₙ bedeutet und E die für Formel (I) definierte Bedeutung hat, wobei die Abgangsgruppe X ausgeschlossen ist,
(b) einem Cyclopentadienidanionenaquivalent oder einem substituierten Cyclopentadienidanionenäquivalent
(c) nucleophile Ringöffnung des cyclopentadienylsubstituierten Oxetanderivats mit MetEₙ, worin Met ein Element der ersten, zweiten oder dritten Hauptgruppe des Periodensystems der Elemente ist, n die maximale formale Wertigkeit von Met in der Verbindung MetEₙ bedeutet und E die für Formel (I) definierte Bedeutung hat, wobei die Abgangsgruppe X ausgeschlossen ist,
(d) Gegebenenfalls Modifizierung der gebildeten OH-Funktion zur Gruppe T,
(e) gegebenenfalls Substitution von RO- in der Gruppe T durch E.

10. Verfahren zur Herstellung chiraler, funktionalisierter tripodaler Cyclopentadienderivate, gemäß Anspruch 9, dadurch gekennzeichnet, daß die Substituenten E bei (a) und (c) verschieden sind.

11. Verwendung von tripodalen Cyclopentadienderivaten gemäß den Ansprüchen 1 bis 5 oder deren konjugiertem Anion als Liganden in Metallkomplexen.

12. Verwendung von tripodalen Cyclopentadienderivaten gemäß den Ansprüchen 1 bis 5 oder deren konjugiertem Anion als Liganden in Katalysatorsystemen.

13. Tripodmetallkomplexe der allgemeinen Formel (V)
LₙM(Tₚ)ₘ (V)
worin
M ein Übergangsmetall oder ein Hauptgruppenmetall des Periodensystems der Elemente
Tp ein Cyclopentadienderivat der allgemeinen Formel (I) 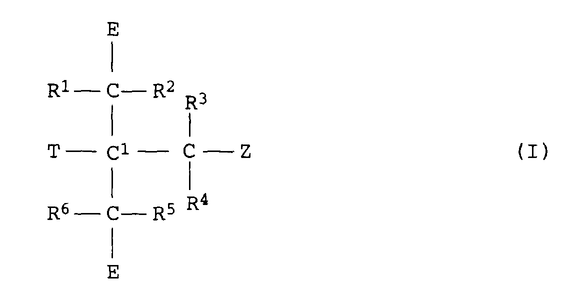
oder dessen konjugiertes Anion ist,
E gleich oder verschieden ist und für -N(R) (R), -P(R) (R), -As(R) (R), -Sb(R) (R), -OR, -SR, -SeR, -TeR, wobei R gleich oder verschieden ist und Wasserstoff- einen C₁- bis C₂₀-kohlenstofforganischen oder C₁- bis C₃₀-siliciumorganischen Rest bedeutet, oder E für eine Abgangsgruppe X steht und
R¹, R², R³, R⁴, R⁵, R⁶ gleich oder verschieden ist und Wasserstoff einen C₁- bis C_{2O}- kohlenstofforganischen oder C₁- bis C_{3O}-siliciumorganischen Rest bedeutet,
Z für den Cyclopentadienylrest oder eine substituierte Cyclopentadienylstruktureinheit steht und
T Wasserstoff einen C₁- bis C₂₀-kohlenstofforganischen oder C₁- bis C₃₀-siliciumorganischen Rest bedeutet oder für eine Gruppe E-Y- steht, worin E für -N(R) (R), -P(R) (R), -As(R) (R), -Sb (R) (R), -OR, -SR, -SeR, -TeR oder eine Abgangsgruppe X steht, wobei R gleich oder verschieden ist und Wasserstoff einen C₁- bis C_{2O}-kohlenstofforganischen oder C₁- bis C_{3O}- siliciumorganischen Rest bedeutet und worin Y eine C₁- bis C_{2O}-organische Gruppe ist, die E mit C¹ verbindet.
L ein formal anionischer oder neutraler Ligand, oder gleiche oder verschiedene derartige Liganden
n eine ganze Zahl von 0 bis 7,
m eine ganze Zahl von 1 bis 8
bedeutet.

14. Verwendung von Tripodmetallkomplexen gemäß Anspruch 13 zur stöchiometrischen oder katalytischen Kohlenstoff-Kohlenstoff-Bindungsverknüpfung oder Hydrierung.
